# EUROPEAN PATENT SPECIFICATION

(11) **EP 1 282 684 B1**
(45) Date of publication and mention of the grant of the patent: **23.09.2009**
(21) Application number: 01927344.0
(22) Date of filing: 25.04.2001
(51) Int. Cl.: C12N 1/00, C12N 5/10, C12N 15/63, C08B 37/08, A61K 31/715

(54) **CHONDROITIN SYNTHASE GENE AND METHODS OF MAKING AND USING SAME**
CHONDROITIN SYNTHASEGEN UND VERFAHREN ZUR DESSEN HERSTELLUNG UND ANWENDUNG
GENE DE LA CHONDROITINE SYNTHASE ET METHODES DE FABRICATION ET D'UTILISATION CORRESPONDANTES

(30) Priority: 25.04.2000 US 199538 P
(43) Date of publication of application: 12.02.2003
(73) Proprietor: The Board of Regents of the University of Oklahoma, Norman, OK 73019 (US)
(72) Inventor: DeAngelis, Paul L., Edmond, OK 73034 (US)
(74) Representative: Ebner von Eschenbach, Jennifer
(86) International application number: PCT/US2001/013395
(87) International publication number: WO 2001/080810

(56) References cited:
- WO-A-99/51265
- WO-A2-00/27437
- US-A- 4 585 754
- DEANGELIS ET AL.: 'Identification and molecular cloning of a chondroitin synthase from Pasteurella multocida type F' J. BIOLOGICAL CHEMISTRY vol. 275, no. 31, 04 August 2000, pages 24124 - 24129, XP002163608
- LIDHOLT ET AL.: 'Biosynthesis of the escherichia coli K4 capsule polysaccharide' J. BIOLOGICAL CHEMISTRY vol. 272, no. 5, 31 January 1997, pages 2682 - 2687, XP002908337
- CHUNG ET AL.: 'The capsule biosynthetic locus of Pasteurella multocida A:1' FEMS MICROBIOLOGY LETTERS vol. 186, 1998, pages 289 - 296, XP002921439

## Description

### BACKGROUND OF THE INVENTION

### 1. Field of the Invention

The present invention relates to a chondroitin synthase gene and methods of using same. In more particular, but not by way of limitation, the present invention relates to a chondroitin synthase gene from *Pasteurella multocida* and methods of using same. Additionally, the present invention relates to the use of unsulfated chondroitin and its preparation, as well as conversion into modified versions such as dermatan sulfate and chondroitin sulfate polymers.

### 2. Background Information Relating to the Invention

Glycosaminoglycans [GAGs] are long linear polysaccharides consisting of disaccharide repeats that contain an amino sugar and are found in most animals. Chondroitin [β(1, 4)GlcUA-β(1, 3)GalNAc]ₙ, heparin/heparan [α1 ,4)GlcUA-[β(1, 4)GlcNAC]ₙ, and hyaluronan [β(1, 4)GlcUA-β(1, 3)GlcNAc]ₙ are the three most prevalent GAGs found in humans. Chondroitin and heparin typically have n = 20 to 100, while hyaluronan typically has n = 10³. Chondroitin and heparin are synthesized as glycoproteins and are sulfated at various positions in vertebrates. Hyaluronan is not sulfated in vertebrates. A substantial fraction of the GlcUA residues of heparin and chondroitin are epimerized to form iduronic acid. "

Many lower animals possess these same GAGs or very similar molecules. GAGs play both structural and recognition roles on the cell surface and in the extracellular matrix. By virtue of their physical characteristics, namely a high negative charge density and a multitude of polar hydroxyl groups, GAGs help hydrate and expand tissues. Numerous proteins bind selectively to one or more of the GAGs. Thus the proteins found on cell surfaces or the associated extracellular matrices (*e.g.* CD44, collagen, fibronectin) of different cell types may adhere or interact via a GAG intermediate. Also GAGs may sequester or bind certain proteins (*e.g.* growth or coagulation factors) to cell surfaces.

Certain pathogenic bacteria produce an extracellular polysaccharide coating, called a capsule, which serves as a virulence factor. In a few cases, the capsule is composed of GAG or GAG-like polymers. As the microbial polysaccharide is identical or very similar to the host GAG, the antibody response is either very limited or non-existent. The capsule is thought to assist in the evasion of host defenses such as phagocytosis and complement. Examples of this clever strategy of molecular camouflage are the production of an authentic HA polysaccharide by Gram-negative Type A *Pasteurella multocida* and Gram-positive Group A and C *Streptococcus.* The HA capsule of these microbes increases virulence by 10² to 10³-fold as measured by LD₅₀ values, the number of colony forming units that will kill 50% of the test animals after bacterial challenge.

The invasiveness and pathogenicity of certain *E. coli* strains has also been attributed to their polysaccharide capsules. Two *Escherichia coli* capsular types, K4 and K5, make polymers composed of GAG-like polymers. The *E. coli* K4 polymer is an unsulfated chondroitin backbone decorated with fructose side-branches on the C3 position of the GlcUA residues. The K5 capsular material is a polysaccharide, called heparosan, identical to mammalian heparin except that the bacterial polymer is unsulfated and there is no epimerization of GlcUA to iduronic acid.

The studies of GAG biosynthesis have been instrumental in understanding polysaccharide production in general. The HA synthases were the first GAG glycosyltransferases to be identified at the molecular level. These enzymes utilize UDP-sugar nucleotide substrates to produce large polymers containing thousands of disaccharide repeats. The genes encoding bacterial, vertebrate, and viral HAS enzymes have been cloned. In all these cases, expression studies demonstrated that transformation with DNA encoding a single HAS polypeptide conferred the ability of foreign hosts to synthesize HA. Except for the most recent HAS to be identified, *P. multocida* pmHAS, these proteins have similar amino acid sequences and predicted topology in the membrane. Two classes of HASs have been proposed to exist based on these structural differences as well as potential differences in reaction mechanism

The biochemical study of chondroitin biosynthesis in vertebrates was initiated in the 1960s. Only recently have the mammalian enzymes for elongation of the polysaccharide backbone of chondroitin been tentatively identified by biochemical means. An 80-kDa GlcUA transferase found in vertebrate cartilage and liver was implicated in the biosynthesis of the chondroitin backbone by photoaffinity labeling with an azidoUDP-GlcUA probe. A preparation from bovine serum with the appropriate GalNAc- and GlcUA-transferase activities *in vitro* wasobtained by conventional chromatography, but several bands on SDS polyacrylamide gels (including a few migrating ~80 kDa) were observed.

Chondroitin polysaccharide [(beta-1,3-GalNAc-beta-1,4-GlcUA)ₙ; n=~10-2000) has use as a hyaluronan (HA) polysaccharide substitute in medical or cosmetic applications. Both chondroitin and hyaluronan form viscoelastic gels (suitable for eye or joint applications) or hydrophilic, hygroscopic materials (suitable for moisturizer or wound dressings). Unmodified or underivatized chondroitin is not known to exist or, if present, in very small quantities in the human body. The main advantage is that byproducts of natural HA degradation (by shear, enzyme, radical or oxidation processes) have certain biological activities with respect to vascularization, angiogenesis, cancer, tissue modulation, but similar byproducts of chondroitin (in the proposed unsulfated, unmodified state) may not have the same biological activity. The chondroitin polymers are more inert, loosely speaking, than the analogous HA molecule. Chondroitin from either *P. multocida* Type F or a recombinant host containing the *Pasteurella*-derived or *Pasteurella*-like synthase gene will serve as an alternative biomaterial with unique properties.

With respect to related microbial GAG synthases other than the HASs, only the *E*. *coli* K5 glycosyltransferases, that synthesizes heparosan have been identified by genetic and biochemical means. In contrast to the HASs, it appears that two proteins, KfiA and KfiC, are required to transfer the sugars of the disaccharide repeat to the growing polymer chain. The chondroitin-backbone synthesizing enzymes of E. *coli* K4 have been enzymatically characterized, but the genes encoding the relevant glycosyltransferases have not yet been identified.

Many *P. multocida* isolates produce GAG or GAG-like molecules as assessed by enzymatic degradation and removal of the capsule of living bacterial cells. Type A *P. multocida,* the major fowl cholera pathogen, makes a capsule that is sensitive to hyaluronidase. Subsequent NMR structural studies of capsular extracts confirmed that HA was the major polysaccharide present. A specific HA-binding protein, aggrecan, also interacts with HA from Type A *P. multocida.* Two other distinct *P. multocida* types, a swine pathogen, Type D, and a minor fowl cholera pathogen, Type F, produce polymers that are chondroitin or chondroitin-like based on the observation that their capsules are degraded by *Flavobacterium* chondroitin AC lyase. After enzymatic removal of the capsule, both types were more readily phagocytosed by neutrophils in vitro. The capsule of Type D cells, but not Type F cells, is also reported to be degraded by heparinase III, suggesting a heparin-type molecule is present, too.

We have analyzed the monosaccharide composition of the *P. multocida* Type F polysaccharide and used the DNA sequence information of the Type A HA biosynthesis locus to obtain the homologous region from the Type F chromosome. From this research we have identified a chondroitin synthase, named pmCS (*P. multocida* Chondroitin Synthase), the first chondroitin synthase to be identified and molecularly cloned from any source. Interestingly, a single polypeptide is responsible for the copolymerization of the GlcUA and GalNAc sugars. We also identified the Type F Capsular polymer as an unsulfated chondroitin polymer. We also identify organisms with the chondroitin synthase gene (Type F *P. multocida*) as new sources of unsulfated chondroitin polymer.

WO 99/51265 discloses in the therein identified SEQ ID NO:3 an amino acid sequence of *P. multocida* chondroitin synthase having 86.8% identity (87.5% ungapped) in a 972 amino acid overlap (1-965:1-972) with that of herein identified SEQ ID NO:4.

### SUMMARY OF THE INVENTION

The present invention provides a purified nucleic acid segment consisting of a coding region encoding enzymatically active soluble chondroitin synthase, wherein the purified nucleic acid segment is selected from the group consisting of:
(A) a purified nucleic acid segment corresponding to residues 45 to 704 of SEQ ID NO:3;
(B) a purified nucleic acid segment corresponding to residues 75 to 704 of SEQ ID NO:3; and
(C) a purified nucleic acid segment corresponding to residues 1 to 704 of SEQ ID NO:3.

Further aspects of the present invention are as defined in the appended claims and include recombinant vectors comprising such nucleic acid segments; recombinant host cells comprising such recombinant vector; and methods of producing a chondroitin polymer *in vitro* or *in vivo* utilising the above-described chondroitin synthase.

### BRIEF DESCRIPTION OF THE SEVERAL VIEWS OF THE DRAWINGS

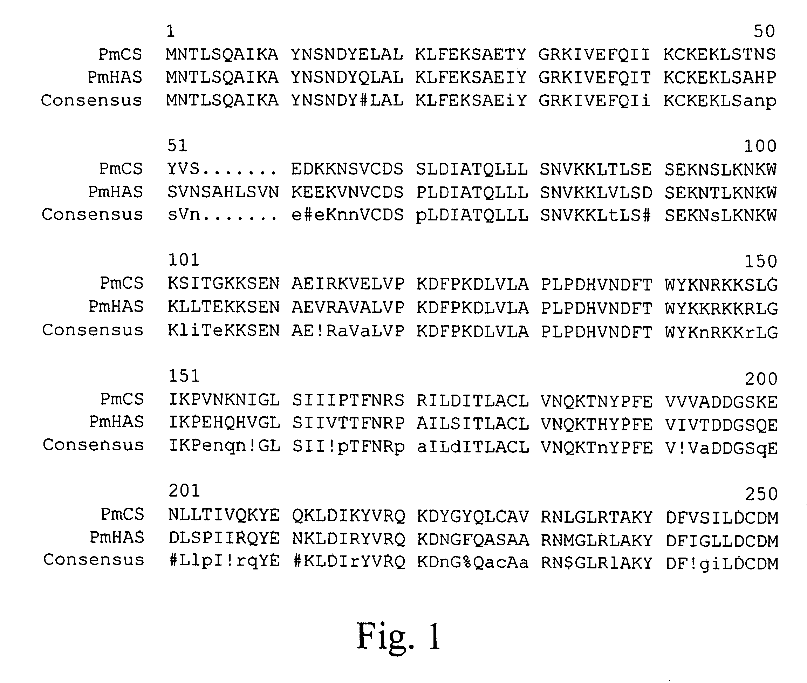

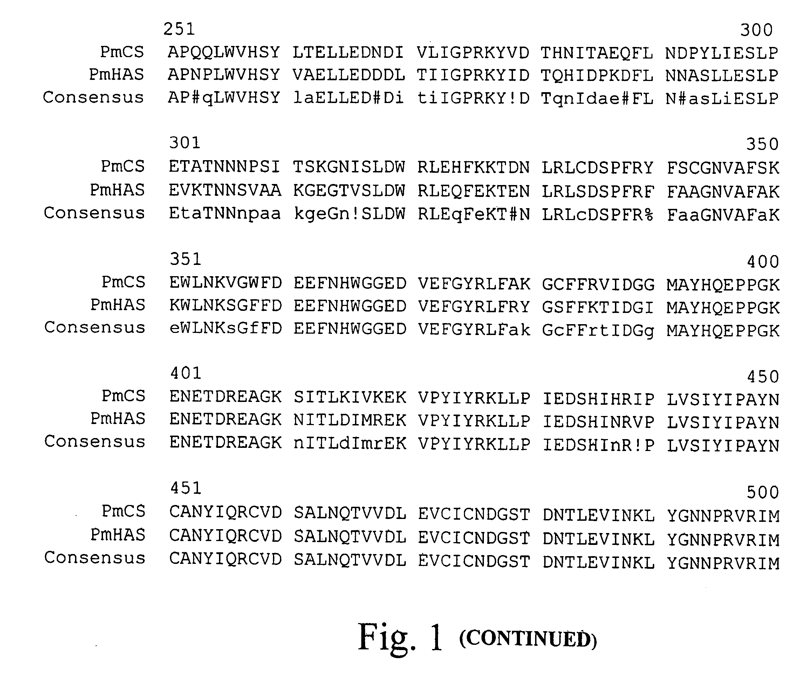

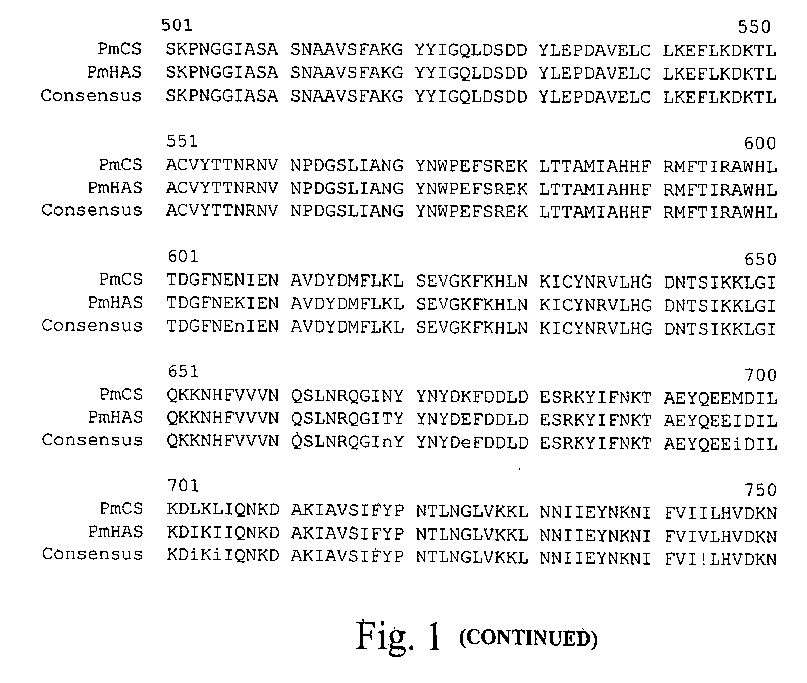

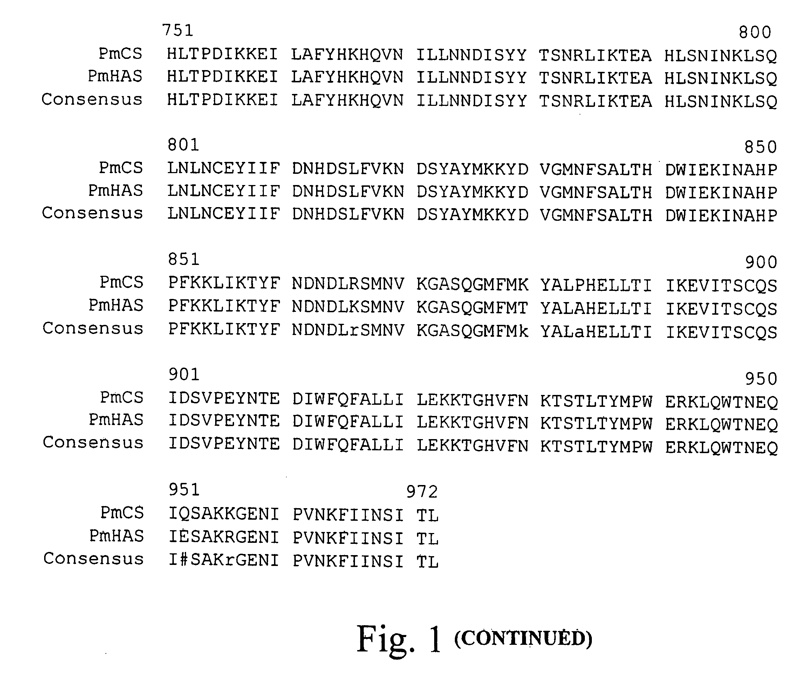

Fig. 1. Sequence Alignment of pmCS and pmHAS. The two *Pasteurella* GAG synthases are highly homologous. Identical residues are denoted with the *hyphen*. The numbering scheme corresponds to the slightly longer pmHAS sequence. The putative A1 (residues 161-267) and A2 (residues 443-547) domains correspond to regions important for hexosamine transferase or for glucuronic acid transferase activity, respectively. Most sequence differences are found in the amino-terminal half of the polypeptides. The "consensus sequence" between pmCS and pmHAS depicts identical residues and similar residues (Consensus symbols denoted as: ! is anyone of IV; $ is anyone of LM; % is anyone of FY; # is anyone of NDQEBZ ). Multalin version 5.4.1 Multiple sequence alignment with hierarchical clustering F. CORPET, 1988, Nucl. Acids Res., 16 (22), 10881-10890 Symbol comparison table: blosum62 Gap weight: 12 Gap length weight: 2 Consensus levels: high=90% low=50%

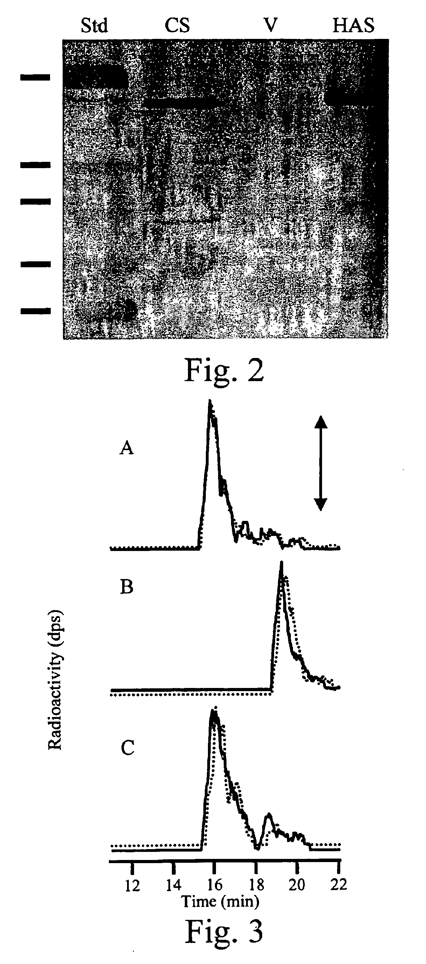

Fig. 2. Western Blot Analysis of Truncated Recombinant *PASTEURELLA* GAG Synthases. Immunoreactive bands at the predicted size of 80 kDa correspond to pmCS¹⁻⁷⁰⁴**(CS)** or pmHAS¹⁻⁷⁰³**(HAS).** No similar band is seen for the vector control **(V).** Prestained standards **(Stds)** are shown for size comparison.
Fig. 3. Gel Filtration Analysis of Radiolabeled Polymer Synthesized *in vitro.* The pmCS¹⁻⁷⁰⁴ extract (1mg total protein) was incubated with chondroitin acceptor oligosaccharide (5 µg), UDP-[¹⁴C]GlcUA and UDP-[3^{H}]GalNAc (580 µM, 0.16 µCi each) in a reaction volume of 200 µl for 30 min. The reaction product was split into five aliquots and treated with various GAG glycosidases as described in *Experimental Procedures.* Portions (60%) of the samples were then analyzed on the PolySep column (calibration elution times in minutes: void volume, 12.7; 580 kDa dextran, 15.4 kDa dextran, 16.0, totally included volume, 19.3 min). Radioactivity (¹⁴C, *solid line*; ³H, *dotted line*) measured by the in-line detector is presented as disintegrations per second (dps). The *double-headed arrow* corresponds to a response of 20 dps. **A,** untreated polymer, peak 15.9 min; **B,** *Flavobacterium* chondroitinase AC lyase-treated polymer, peak, 19.2 min; **C**, HA lyase-treated polymer, peak 15.9 min. The polymer peak with a size of ~100 to 400 kDa contained both radiolabeled sugars at a 1:1 ratio and was degraded only by the appropriate enzyme, chondroitin AC lyase.

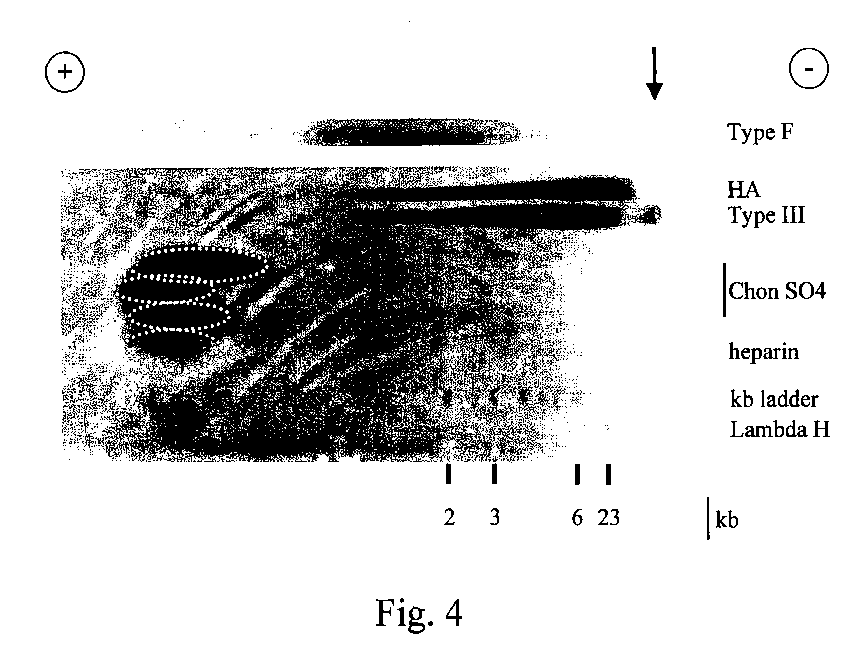

Fig. 4. Gel Electrophoresis Analysis of Polysaccharides. Various polymers were separated by their mass/charge ratio on 0.8% agarose gels in a 1X TAE (tris acetate EDTA) buffer sytem. About 9 ug of polymer were loaded per lane and separated using a field of 0.8 volts/cm. Lanes: Type F, native polymer from *Pasteurella multocida* Type F; HA, hyaluronic acid; Type III, *Streptococcus pneumoniae* type 3 capsular polymer; Chon SO4, Chondroitin sulfate C,B,A - from left to right [note: marked in white dotted circle]; heparin, porcine heparin; kb ladder, kilobase DNA ladder standard; Lambda H, HindIII digest of lambda virus DNA standard. Sizes of select DNA standards are marked in kilobases (kb). After electrophoresis, the gel was stained with the dye Stains-All (Sigma; 0.05% in 50% ethanol, w/v) for 2 hours, then destained in water. Type F polymer runs slower than chondroitin sulfate polymers because it is larger and not as highly charged (missing the extra charges of sulfates). The staining color for HA, Type III and Type F polymers is blue, but chondroitin sulfate stains purple. The Type F polysaccharide is a novel large unsulfated chondroitin chain, the first report of this polymer.

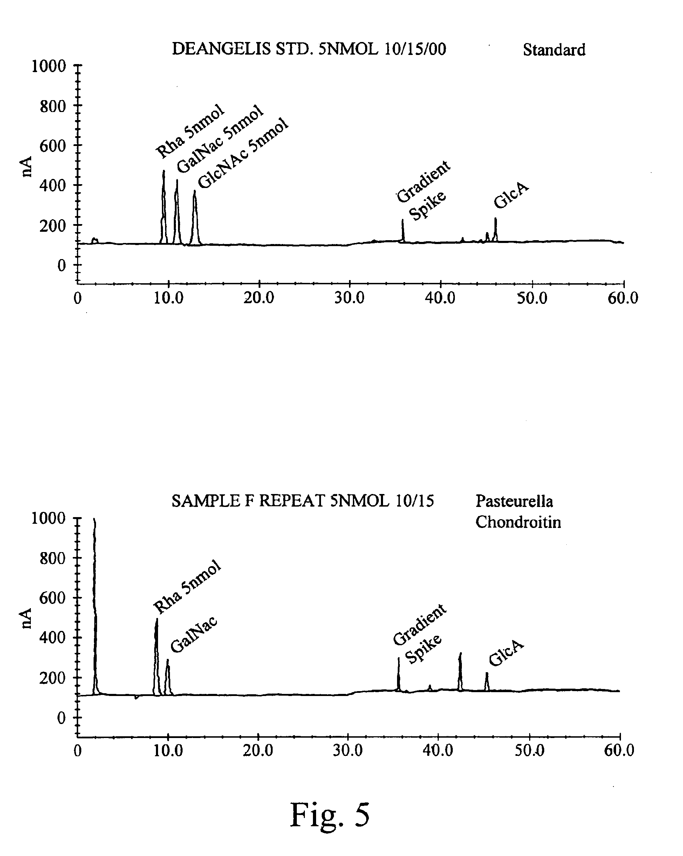

Fig. 5. Monosaccharide Analysis of Type F Polymer. The examples of the actual chromatogram used to generate the sugar composition data in Table 2 are shown here. The profiles graph the amperometric response (y-axis signifying the relative amount of sugar eluting from column; units nA = nanoamperes) versus elution time (x-axis signifying the distinct monosaccharides; units = minutes). All injected samples (containing about 5 nanomoles of each sugar component) were spiked with an internal standard, rhamnose (Rha), to assess recovery and the column performance. The "DeAngelis Standard" contains a mixture of GalNAc, GlcNAc, and GlcUA that were subjected to hydrolysis. The "Sample F" is a hydrolysate of Type F polymer. The analysis shows that the Type F polymer is composed of only GalN (hydrolysis removes the acetyl group converting GalNAc into GAlN) and GlcUA monosaccharides.

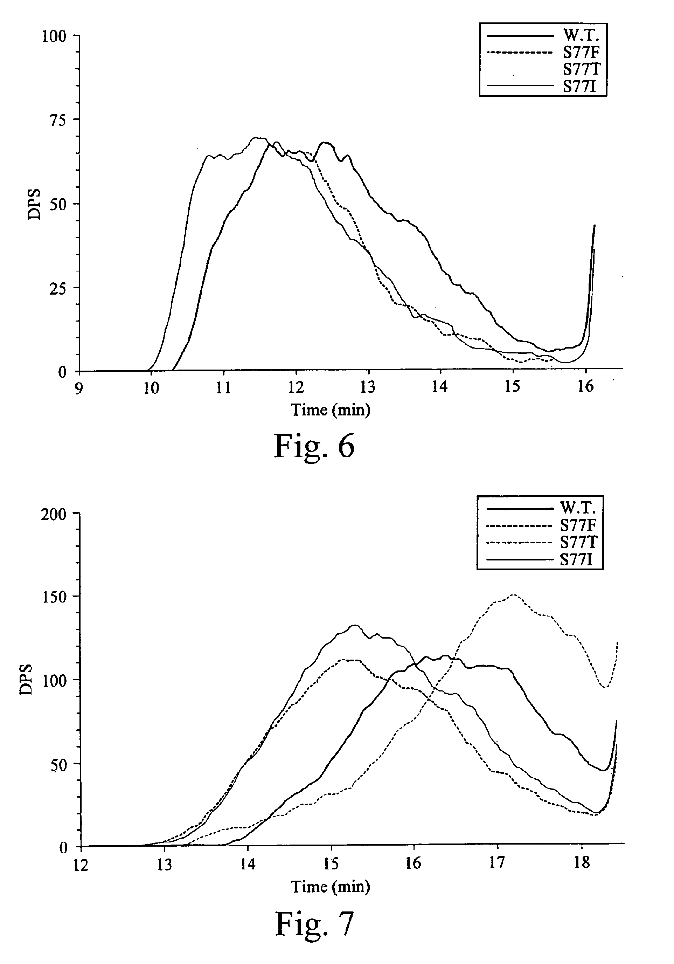

Fig. 6. HA Product Size Analysis of xlHAS1-Ser77 Mutants. The various enzymes were assayed for 5 minutes and the HA polymer products were separated by high performance gel filtration. Depending on the nature of the substituting amino acid residue at position 77, either larger or smaller HA products were formed in comparison to HA products polymerized by the wild-type enzyme. Only two mutants, Ser77Ile (larger HA) and Ser77Thr (smaller HA), and the wild-type synthase are shown. 5 min products separated on a PolySep-4000 column. For comparison, the 580-kDa dextran standard eluted at 12.5 min or 16.8 min on the 4000 or 6000 column, respectively.
Fig. 7. HA Product Size Analysis of xlHAS1-Ser77 Mutants. The various enzymes were assayed for 30 minutes and the HA polymer products were separated by high performance gel filtration. Depending on the nature of the substituting amino acid residue at position 77, either larger or smaller HA products were formed in comparison to HA products polymerized by the wild-type enzyme. Only two mutants, Ser77Ile (larger HA) and Ser77Thr (smaller HA), and the wild-type synthase are shown. 30 min products separated on a PolySep-6000 column. For comparison, the 580-kDa dextran standard eluted at 12.5 min or 16.8 min on the 4000 or 6000 column, respectively.

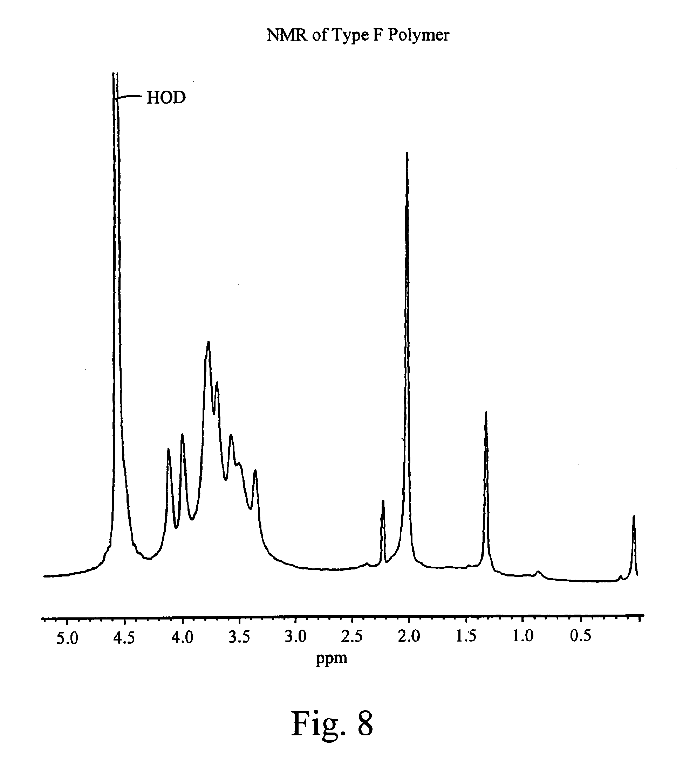

Fig. 8. NMR Analysis - Type F polysaccharide (170 ug uronic acid based on carbazole reaction) was exchanged into deuterated water (D₂O) and the proton spectrum (H-NMR) was acquired at 45° C at 500 MHz. The chemical shifts (ppm) of the peaks are consistent with an unsulfated chondroitin polymer.

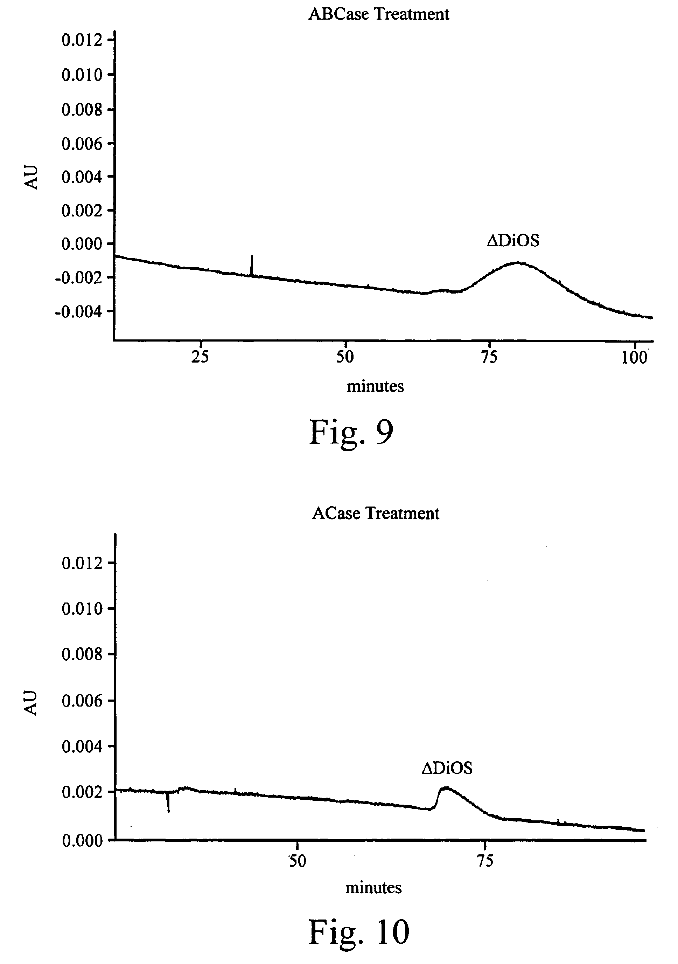

Fig. 9. Disaccharide Composition Analysis - Samples were dissolved in 10 µl of 50 mM Tris-HCl and 60. mM sodium acetate buffer, pH 8, and 20 mU Chondroitinase ABC was added separately. The digestions were kept in a 37° C water bath overnight. The enzymes were deactivated by boiling for 2 min. The experiments were performed with a capillary electrophoresis P/ACE 5500 System (Beckman Instruments, Fullerton, CA) at a constant capillary temperature of 18° C with a potential of -22 kV by UV absorbance at 232 nm. The electropherograms were acquired using the system Gold software package (Beckman Instruments, Fullerton, CA). Separation and analysis were carried out in a reversed polarity mode using a fused silica (externally coated except where the tube passed through the detector) capillary tube (50 µm inner diameter, 360 µm outer diameter, 57 cm long, and 49 cm effective length). Prior to every run, the capillary was conditioned with 0.5M NaOH (1 min, 0,14 MPa [20 psi]) and rinsed (1 min, 0,14MPa [20 psi]) with separation buffer (20 mM H₃PO₄ adjusted to pH 3.5 with saturated dibasic sodium phosphate). Samples were applied by pressure injection 25 s at 0.003 MPa (0.5 psi). Standards of all potential chondroitin oligosaccharides were used to calibrate the capillary. The unsulfated disaccharide, ADiOS, was observed in digests of Type F polymer. Thus, the native polymer of Type F is an unsulfated chondroitin.
Fig. 10. Disaccharide Composition Analysis - Samples were dissolved in 10 µl of 50 mM Tris-HCl and 60 mM sodium acetate buffer, pH 8, and 20 mU Chondroitinase AC was added separately. The digestions were kept in a 37° C water bath overnight. The enzymes were deactivated by boiling for 2 min. The experiments were performed with a capillary electrophoresis P/ACE 5500 System (Beckman Instruments, Fullerton, CA) at a constant capillary temperature of 18° C with a potential of -22 kV by UV absorbance at 232 nm. The electropherograms were acquired using the system Gold software package (Beckman Instruments, Fullerton, CA). Separation and analysis were carried out in a reversed polarity mode using a fused silica (externally coated except where the tube passed through the detector) capillary tube (50 µm inner diameter, 360 µm outer diameter, 57 cm long, and 49 cm effective length). Prior to every run, the capillary was conditioned with 0.5M NaOH (1 min, 0.14 MPa [20 psi]) and rinsed (1 min, 0.14 MPa [20 psi]) with separation buffer (20 mM H₃PO₄ adjusted to pH 3.5 with saturated dibasic sodium phosphate). Samples were applied by pressure injection 25 s at 0.003 MPa (0.5 psi). Standards of all potential chondroitin oligosaccharides were used to calibrate the capillary. The unsulfated disaccharide ΔDiOS, was observed in digests of Type F polymer. Thus, the native polymer of Type F is an unsulfated chondroitin.

### DETAILED DESCRIPTION OF THE INVENTION

Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for purpose of description and should not be regarded as limiting.

As used herein, the term "nucleic acid segment" and "DNA segment" are used interchangeably and refer to a DNA molecule which has been isolated free of total genomic DNA of a particular species. Therefore, a "purified" DNA or nucleic acid segment as used herein, refers to a DNA segment which contains a Chondroitin Synthase ("CS") coding sequence yet is isolated away from, or purified free from, unrelated genomic DNA, for example, total *Pasteurella multocida* or, for example, mammalian host genomic DNA. Included within the term "DNA segment", are DNA segments and smaller fragments of such segments, and also recombinant vectors, including, for example, plasmids, cosmids, phage, viruses, and the like.

Similarly, a DNA segment comprising an isolated or purified pmCS (*Pasteurella multocida* Chondroitin Synthase) gene refers to a DNA segment including Chondroitin Synthase coding sequences isolated substantially away from other naturally occurring genes or protein encoding sequences. In this respect, the term "gene" is used for simplicity to refer to a functional protein, polypeptide or peptide encoding unit. As will be understood by those in the art, this functional term includes genomic sequences, cDNA sequences or combinations thereof. "Isolated substantially away from other coding sequences" means that the gene of interest, in this case pmCS, forms the significant part of the coding region of the DNA segment, and that the DNA segment does not contain large portions of naturally-occurring coding DNA, such as large chromosomal fragments or other functional genes or DNA coding regions. Of course, this refers to the DNA segment as originally isolated, and does not exclude genes or coding regions later added to, or intentionally left in the segment by the hand of man.

Due to certain advantages associated with the use of prokaryotic sources, one will likely realize the most advantages upon isolation of the CS gene from *Pasteurella multocida.* One such advantage is that, typically, eukaryotic enzymes may require significant post-translational modifications that can only be achieved in a eukaryotic host. This will tend to limit the applicability of any eukaryotic CS gene that is obtained. Moreover, those of ordinary skill in the art will likely realize additional advantages in terms of time and ease of genetic manipulation where a prokaryotic enzyme gene is sought to be employed. These additional advantages include (a) the ease of isolation of a prokaryotic gene because of the relatively small size of the genome and, therefore, the reduced amount of screening of the corresponding genomic library and (b) the ease of manipulation because the overall size of the coding region of a prokaryotic gene is significantly smaller due to the absence of introns.
Furthermore, if the product of the Chondroitin Synthase gene (i.e., the enzyme) requires posttranslational modifications or cofactors, these would best be achieved in a similar prokaryotic cellular environment (host) from which the gene was derived.

Preferably, DNA sequences in accordance with the present invention will further include genetic control regions which allow the expression of the sequence in a selected recombinant host. Of course, the nature of the control region employed will generally vary depending on the particular use (e.g., cloning host) envisioned.

Disclosed herein are isolated DNA segments and recombinant vectors incorporating DNA sequences which encode a Chondroitin Synthase gene such as pmCS. In the case of pmCS, the isolated DNA segments and recombinant vectors incorporating DNA sequences which include within their amino acid sequences an amino acid sequence in accordance with SEQ ID NO:2 or 4. Moreover, in other particular cases, the disclosure concerns isolated DNA segments and recombinant vectors incorporating DNA sequences which encode a gene that includes, within its amino acid sequence the amino acid sequence of an Chondroitin Synthase gene or DNA, and in particular to an Chondroitin Synthase gene or cDNA, corresponding to *Pasteurella multocida* Chondroitin Synthase - pmCS. For example, where the DNA segment or vector encodes a full length Chondroitin Synthase protein, or is intended for use in expressing the Chondroitin Synthase protein, the sequences are those which are essentially as set forth in SEQ ID NO:2 or 4.

The original sequences (SEQ ID NOS: 1 and 2) differ from the corrected sequences (SEQ ID NOS: 2 and 4) because, after more extensive sequencing of the plasmid template encoding the original functional pmCS gene, we found a few mistakes made by the sequencing technician in the original DNA sequence. Basically, certain regions of the pmCS gene are very difficult to sequence accurately. Typically, one can obtain a sequence read length of 200-700 bases with a non-problematic sequence. However, in the case of pmCS gene, there were certain places where no more than 20 to 50 bases could be read, and even those were difficult. The two reasons for sequencing problems are usually due to: (1) the template, which should remain single-stranded during the reaction, forming double-stranded regions or loops in the problematic template that cause the sequencing polymerase to fall off the template prematurely, and (2)the sequencing product forms loops structures or twisted forms while running on the electrophoresis gel that do not run in the desired single-stranded conformation. We have now corrected the sequence to reflect the actual DNA and protein sequences of pmCS.

SEQ ID NO: 1 and 2 have been assigned GenBank Accession No. AF195517.

Nucleic acid segments having chondroitin synthase activity may be isolated by the methods described herein. The term "a sequence essentially as set forth in SEQ ID NO:2 or 4" means that the sequence substantially corresponds to a portion of SEQ ID NO:2 or 4 and has relatively few amino acids which are not identical to, or a biologically functional equivalent of, the amino acids of SEQ ID NO:2 or 4. The term "biologically functional equivalent" is well understood in the art and is further defined in detail herein, as a gene having a sequence essentially as set forth in SEQ ID NO:2 or 4, and that is associated with the ability of prokaryotes to produce chondroitin or a "chondroitin like" polymer or a chondroitin synthase polypeptide.

One of ordinary skill in the art would appreciate that a nucleic acid segment encoding enzymatically active chondroitin synthase may contain conserved or semi-conserved substitutions to the sequences set forth in SEQ ID NOS: 1, 2, 3 or 4.

In particular, the art is replete with examples of practitioners ability to make structural changes to a nucleic acid segment (i.e. encoding conserved or semi-conserved amino acid substitutions) and still preserve its enzymatic or functional activity. See for example: (1) Risler et al. "Amino Acid Substitutions in Structurally Related Proteins. A Pattern Recognition Approach." J. Mol. Biol. 204:1019-1029 (1988) ["... according to the observed exchangeability of amino acid side chains, only four groups could be delineated; (i) Ile and Val; (ii) Leu and Met, (iii) Lys, Arg, and Gln, and (iv) Tyr and Phe."]; (2) Niefind et al. "Amino Acid Similarity Coefficients for Protein Modeling and Sequence Alignment Derived from Main-Chain Folding Anoles." J. Mol. Biol. 219:481-497 (1991) [similarity parameters allow amino acid substitutions to be designed]; and (3) Overington et al. "Environment-Specific Amino Acid Substitution Tables: Tertiary Templates and Prediction of Protein Folds," Protein Science 1:216-226 (1992) ["Analysis of the pattern of observed substitutions as a function of local environment shows that there are distinct patterns..." Compatible changes can be made.]

These references and countless others available to one of ordinary skill in the art, indicate that given a nucleic acid sequence, one of ordinary skill in the art could make substitutions and changes to the nucleic acid sequence without changing its functionality. Also, a substituted nucleic acid segment may be highly identical and retain its enzymatic activity with regard to its unadulterated parent, and yet still fail to hybridize thereto.

One of ordinary skill in the art would also appreciate that substitutions can be made to the pmCS nucleic acid segment listed in SEQ ID NO:3 without deviating outside the scope and claims of the present invention. Standardized and accepted functionally equivalent amino acid substitutions are presented in Table I.

**TABLE I**

| **Amino Acid Group** | **Conservative and Semi-Conservative Substitutions** |
|---|---|
| NonPolar R Groups | Alanine, Valine, Leucine, Isoleucine, Proline, Methionine, Phenylalanine, Tryptophan |
| Polar, but uncharged, R Groups | Glycine, Serine, Threonine, Cysteine, Asparagine, Glutamine |
| Negatively Charged R Groups | Aspartic Acid, Glutamic Acid |
| Positively Charged R Groups | Lysine, Arginine, Histidine |

Further disclosed herein is a purified nucleic acid segment that encodes a protein in accordance with SEQ ID NO:2 or 4, further defined as a recombinant vector. As used herein, the term "recombinant vector" refers to a vector that has been modified to contain a nucleic acid segment that encodes a Chondroitin Synthase protein, or fragment thereof. The recombinant vector may be further defined as an expression vector comprising a promoter operatively linked to said Chondroitin Synthase encoding nucleic acid segment.

Yet further disclosed herein is a host cell, made recombinant with a recombinant vector comprising a Chondroitin Synthase gene. The preferred recombinant host cell may be a prokaryotic cell. In another embodiment, the recombinant host cell is a eukaryotic cell. As used herein, the term "engineered" or "recombinant" cell is intended to refer to a cell into which a recombinant gene, such as a gene encoding Chondroitin Synthase, has been introduced. Therefore, engineered cells are distinguishable from naturally occurring cells which do not contain a recombinantly introduced gene. Engineered cells are thus cells having a gene or genes introduced through the hand of man. Recombinantly introduced genes will either be in the form of a cDNA gene, a copy of a genomic gene, or will include genes positioned adjacent to a promoter not naturally associated with the particular introduced gene.

Where one desires to use a host other than *Pasteurella,* as may be used to produce recombinant chondroitin synthase, it may be advantageous to employ a prokaryotic system such as *E. coli, B. subtilis, Lactococcus sp.,* or even eukaryotic systems such as yeast or Chinese hamster ovary, African green monkey kidney cells, VERO cells, or the like. Of course, where this is undertaken it will generally be desirable to bring the chondroitin synthase gene under the control of sequences which are functional in the selected alternative host. The appropriate DNA control sequences, as well as their construction and use, are generally well known in the art as discussed in more detail hereinbelow.

In preferred embodiments, the chondroitin synthase-encoding DNA segments further include DNA sequences, known in the art functionally as origins of replication or "replicons", which allow replication of contiguous sequences by the particular host. Such origins allow the preparation of extrachromosomally localized and replicating chimeric segments or plasmids, to which chondroitin synthase DNA sequences are ligated. In more preferred instances, the employed origin is one capable of replication in bacterial hosts suitable for biotechnology applications. However, for more versatility of cloned DNA segments, it may be desirable to alternatively or even additionally employ origins recognized by other host systems whose use is contemplated (such as in a shuttle vector).

The isolation and use of other replication origins such as the SV40, polyoma or bovine papilloma virus origins, which may be employed for cloning or expression in a number of higher organisms, are well known to those of ordinary skill in the art. In certain embodiments, the invention may thus be defined in terms of a recombinant transformation vector which includes the chondroitin synthase coding gene sequence together with an appropriate replication origin and under the control of selected control regions.

Thus, it will be appreciated by those of skill in the art that other mean may be used to obtain the Chondroitin Synthase gene or cDNA, in light of the present disclosure. For example, polymerase chain reaction or RT-PCR produced DNA fragments may be obtained which contain full complements of genes or cDNAs from a number of sources, including other strains of *Pasteurella* or from eukaryotic sources, such as cDNA libraries. Virtually any molecular cloning approach may be employed for the generation of DNA fragments in accordance with the present invention. Thus, the only limitation generally on the particular method employed for DNA isolation is that the isolated nucleic acids should encode a biologically functional equivalent chondroitin synthase.

Once the DNA has been isolated it is ligated together with a selected vector. Virtually any cloning vector can be employed to realize advantages in accordance with the invention. Typical useful vectors include plasmids and phages for use in prokaryotic organisms and even viral vectors for use in eukaryotic organisms. Examples include pKK223-3, pSA3, recombinant lambda, SV40, polyoma, adenovirus, bovine papilloma virus and retroviruses. However, it is believed that particular advantages will ultimately be realized where vectors capable of replication in both *Lactococcus* or *Bacillus* strains and *E. coli* or *P. multocida* are employed.

Vectors such as these, exemplified by the pSA3 vector of Dao and Ferretti or the pAT19 vector of Trieu-Cuot, et al., allow one to perform clonal colony selection in an easily manipulated host such as *E. coli,* followed by subsequent transfer back into a food grade *Lactococcus* or *Bacillus* strain for production of chondroitin. These are benign and well studied organisms used in the production of certain foods and biotechnology products -- otherwise known in the art as GRAS (Generally Regarded As Safe). GRAS organisms are advantageous in that one can augment the *Lactococcus* or *Bacillus* strain's ability to synthesize chondroitin through gene dosaging (i.e., providing extra copies of the HA synthase gene by amplification) and/or the inclusion of additional genes to increase the availability of the chondroitin precursors UDP-GlcUA and UDP-GalNAc. These precursors are made by the action of UDP-glucose dehydrogenase and UDP-GlcNAc/UDP-GalNAc epimerase, respectively.
The inherent ability of a bacterium to synthesize chondroitin can also be augmented through the formation of extra copies, or amplification, of the plasmid that carries the chondroitin synthase gene. This amplification can account for up to a 10-fold increase in plasmid copy number and, therefore, the Chondroitin Synthase gene copy number.

Another procedure that would further augment Chondroitin Synthase gene copy number is the insertion of multiple copies of the gene into the plasmid. Another technique would include integrating the Chondroitin Synthase gene into chromosomal DNA. This extra amplification would be especially feasible, since the Chondroitin Synthase gene size is small. In some scenarios, the chromosomal DNA-ligated vector is employed to transfect the host that is selected for clonal screening purposes such as *E. coli* or *Bacillus,* through the use of a vector that is capable of expressing the inverted DNA in the chosen host. In certain instances, especially to confer stability, genes such as the chondroitin synthase gene, may be integrated into the chromosome in various positions in an operative fashion. Unlike plasmids, integrated genes do not need selection pressure for maintenance of the recombinant gene.

Where a eukaryotic source such as dermal or synovial fibroblasts or rooster comb cells is employed, one will desire to proceed initially by preparing a cDNA library. This is carried out first by isolation of mRNA from the above cells, followed by preparation of double stranded cDNA using an enzyme with reverse transcriptase activity and ligation with the selected vector. Numerous possibilities are available and known in the art for the preparation of the double stranded cDNA, and all such techniques are believed to be applicable. A preferred technique involves reverse transcription. Once a population of double stranded cDNAs is obtained, a cDNA library is prepared in the selected host by accepted techniques, such as by ligation into the appropriate vector and amplification in the appropriate host. Due to the high number of clones that are obtained, and the relative ease of screening large numbers of clones by the techniques set forth herein, one may desire to employ phage expression vectors, such as λgt11, λgt12, λGem11, and/or λZAP for the cloning and expression screening of cDNA clones.

The present disclosure also concerns isolated DNA segments and recombinant vectors that include within their sequences a nucleic acid sequence essentially as set forth in SEQ ID NO:1. The term "essentially as set forth in SEQ ID NO:1" is used in the same sense as described above and means that the nucleic acid sequence substantial corresponds to a portion of SEQ ID. NO:1, and has relatively few codons which are not identical, or functionally equivalent, to the codons of SEQ ID NO:1. The term "functionally equivalent codon" is used herein to refer to codons that encode the same amino acid, such as the six codons for arginine or serine, as set forth in Table I, and also refers to codons that encode biologically equivalent amino acids.

It will also be understood that amino acid and nucleic acid sequences may include additional residues, such as additional N- or C-terminal amino acids or 5' or 3' nucleic acid sequences, and yet still be essentially as set forth in one of the sequences disclosed herein, so long as the sequence meets the criteria set forth above, including the maintenance of biological protein activity where protein expression and enzyme activity is concerned. The addition of terminal sequences particularly applies to nucleic acid sequences which may, for example, include various non-coding sequences flanking either of the 5' or 3' portions of the coding region or may include various internal sequences, which are known to occur within genes.

Likewise, deletion of certain portions of the polypeptide can be desirable. For example, functional truncated versions of pmHAS or pmCS missing the carboxyl terminus enhances the utility for *in vitro* use. (Fig. 2, Table 3) The pm HAS¹⁻⁷⁰³ and pmCS¹⁻⁷⁰⁴ are soluble proteins that are easy to purify in contrast to the full-length proteins (972 and 965 residues, respectively). Also, expression level increases greatly as the membrane is not overload. In accordance with the present invention it is also contemplated that the truncated version of pmCS encompasses residues 45-704 and 75-704. These truncated versions are also highly soluble and increases expression; the native membrane proteins are found in low levels and is not soluble without special treatment with detergents.

Allowing for the degeneracy of the genetic code as well as conserved and semi-conserved substitutions, sequences which have between about 40% and about 80%; or more preferably, between about 80% and about 90%; or even more preferably, between about 90% and about 99%; of nucleotides which are identical to the nucleotides of SEQ ID NO:1 will be sequences which are "essentially as set forth in SEQ ID NO:1". Sequences which are essentially the same as those set forth in SEQ ID NO:1 may also be functionally defined as sequences which are capable of hybridizing to a nucleic acid segment containing the complement of SEQ ID NO:1 under standard or less stringent hybridizing conditions. Suitable standard hybridization conditions will be well known to those of skill in the art and are clearly set forth herein. As certain domains and active sites are formed from a relatively small portion of the total polypeptide, these regions of sequence identity or similarity may be present only in portions of the gene.

As well known in the art, most of the amino acids in a protein are present to form the "scaffolding" or general environment of the protein. The actual working parts responsible for the specific desired catalysis are usually a series of small.domains or motifs. Thus a pair of enzymes that possess the same or similar motifs would be expected to possess the same or similar catalytic activity, thus be functionally equivalent. Utility for this hypothetical pair of enzymes may be considered interchangeable unless one member of the pair has a subset of distinct, useful properties. In a similar vein, certain non-critical motifs or domains may be dissected from the original, naturally occurring protein and function will not be affected; removal of non-critical residues does not perturb the important action of the remaining critical motifs or domains. By analogy, with sufficient planning and knowledge, it should be possible to translocate motifs or domains from one enzyme to another polypeptide to confer the new enzyme with desirable characteristics intrinsic to the domain or motif.

The term "standard hybridization conditions" as used herein, is used to describe those conditions under which substantially complementary nucleic acid segments will form standard Watson-Crick base-pairing. A number of factors are known that determine the specificity of binding or hybridization, such as pH, temperature, salt concentration, the presence of agents, such as formamide and dimethyl sulfoxide, the length of the segments that are hybridizing, and the like. When it is contemplated that shorter nucleic acid segments will be used for hybridization, for example fragments between about 14 and about 100 nucleotides, salt and temperature preferred conditions for overnight hybridization will include 1.2-1.8 x HPB at 40-50°C or 5 x SSC at 50AC. Washes in low salt (10mM salt or 0.1 x SSC) are used for stringency and room temperature incubations of 10 - 60 minutes.

Naturally, the present disclosure also encompasses DNA segments which are complementary, or essentially complementary, to the sequence set forth in SEQ ID NOS:1, 2, 3 or 4. Nucleic acid sequences which are "complementary" are those which are capable of base-pairing according to the standard Watson-Crick complementarity rules. As used herein, the term "complementary sequences" means nucleic acid sequences which are substantially complementary, as may be assessed by the same nucleotide comparison set forth above, or as defined as being capable of hybridizing to the nucleic acid segment of SEQ ID NO:1.

The nucleic acid segments described herein regardless of the length of the coding sequence itself, may be combined with other DNA sequences, such as promoters, polyadenylation signals, additional restriction enzyme sites, multiple cloning sites, epitope tags, poly histidine regions, other coding segments, and the like, such that their overall length may vary considerably. It is therefore contemplated that a nucleic acid fragment of almost any length may be employed, with the total length preferably being limited by the ease of preparation and use in the intended recombinant DNA protocol. _

The DNA segments of the present invention encompass biologically functional equivalent Chondroitin Synthase proteins and peptides. Such sequences may arise as a consequence of codon redundancy and functional equivalency which are known to occur naturally within nucleic acid sequences and the proteins thus encoded. Alternatively, functionally equivalent proteins or peptides may be created via the application of recombinant DNA technology, in which changes in the protein structure may be engineered, based on considerations of the properties of the amino acids being exchanged. Changes designed by man may be introduced through the application of site-directed mutagenesis techniques, e.g., to introduce improvements to the enzyme activity or to antigenicity of the Chondroitin Synthase protein or to test Chondroitin Synthase mutants in order to examine chondroitin synthase activity at the molecular level.

Also, specific changes to the Chondroitin Synthase coding sequence can result in the production of chondroitin having a modified size distribution or structural configuration. One of ordinary skill in the art would appreciate that the Chondroitin Synthase coding sequence can be manipulated in a manner to produce an altered chondroitin synthase which in turn is capable of producing chondroitin having differing polymer sizes and/or functional capabilities. For example, the Chondroitin Synthase coding sequence may be altered in such a manner that the chondroitin synthase has an altered sugar substrate specificity so that the chondroitin synthase creates a new chondroitin-like polymer incorporating a different structure via the inclusion of a previously unincorporated sugar or sugar derivative. This newly incorporated sugar could result in a modified chondroitin having different functional properties. As will be appreciated by one of ordinary skill in the art given the Chondroitin Synthase coding sequences, changes and/or substitutions can be made to the Chondroitin Synthase coding sequence such that these desired property and/or size modifications can be accomplished.

Basic knowledge on the substrate binding sites (e.g. the UDP-GlcUA site or UDP-GalNAc site or oligosaccharide acceptor site) of pmCS allows the targeting of residues for mutation to change the catalytic properties of the site. The identity of important catalytic residues of pmHAS, a close homolog of pmCS, have recently been elucidated (Jing & DeAngelis, 2000, Glycobiology vol 10; pp. 883-889). Appropriate changes at or near these residues would allow other UDP-sugars to bind instead of the authentic chondroitin sugar precursors; thus a new, modified polymer is synthesized. Polymer size changes will be caused by differences in the synthase's catalytic efficiency or changes in the acceptor site affinity. Polymer size changes have been made in seHAS and spHAS (Weigel et al, Designer HA) as well as the vertebrate HAS, xlHAS1 (DG42) (Pummill & DeAngelis, unpublished data) by mutating various residues (Figs. 6 and 7). As pmCS is a more malleable, robust enzyme than these other enzymes, similar or superior versions of mutant pmCS which synthesize modified polymers are also possible.

The term "modified structure" as used herein denotes a chondroitin polymer containing a sugar or derivative not normally found in the naturally occurring chondroitin polypeptide. The term "modified size distribution" refers to the synthesis of chondroitin molecules of a size distribution not normally found with the native enzyme; the engineered size could be much smaller or larger than normal.

Various chondroitin products of differing size have.application in the areas of drug delivery and the generation of an enzyme of altered structure can be combined with a chondroitin of differing size. Applications in angiogenesis and wound healing are potentially large if chondroitin polymers of about 10-20 monosaccharides can be made in good quantities. Another particular application for small chondroitin oligosaccharides is in the stabilization of recombinant human proteins used for medical purposes. A major problem with such proteins is their clearance from the blood and a short biological half life. One present solution to this problem is to couple a small molecule shield that prevents the protein from being cleared from the circulation too rapidly. Very small molecular weight chondroitin is well suited for this role and would be nonimmunogenic and biocompatible. Larger molecular chondroitin attached to a drug or protein may be used to target the reticuloendothelial cell system which has endocytic receptors for chondroitin. Large polymers may be used in high concentrations to make gels or viscous solutions with potential for joint lubrications opthaltmic procedures, and cosmetics.

One of ordinary skill in the art given this disclosure would appreciate that there are several ways in which the size distribution of the chondroitin polymer made by the chondroitin synthase could be regulated to give different sizes. First, the kinetic control of product size can be altered by decreasing temperature, decreasing time of enzyme action and by decreasing the concentration of one or both sugar nucleotide substrates. Decreasing any or all of these variables will give lower amounts and smaller sizes of chondroitin product. The disadvantages of these approaches are that the yield of product will also be decreased and it may be difficult to achieve reproducibility from day to day or batch to batch.

Secondly, the alteration of the intrinsic ability of the enzyme to synthesize a large chondroitin product. Changes to the protein can be engineered by recombinant DNA technology, including substitution, deletion and addition of specific amino acids (or even the introduction of prosthetic groups through metabolic processing). Such changes that result in an intrinsically slower enzyme could then allow more reproducible control of chondroitin size by kinetic means. The final chondroitin size distribution is determined by certain characteristics of the enzyme that rely on particular amino acids in the sequence. Among the 10-20% of residues absolutely conserved between streptococcal hyaluronate synthase enzymes, eukaryotic hyaluronate synthase enzymes, and the pmCS, there is a set of amino acids at unique positions that may control or greatly influence the size of the polymer (either hyaluronan or chondroitin) that the enzyme can make.

As shown in Figs. 6 and 7, HA Product Size Analysis of xlHAS1-Ser77 Mutants. The various enzymes were assayed for 5 or 30 minutes and the HA polymer products were separated by high performance gel filtration. Depending on the nature of the substituting amino acid residue at position 77, either larger or smaller HA products were formed in comparison to HA products polymerized by the wild-type enzyme. Only two mutants, Ser77Ile (larger HA) and Ser77Thr (smaller HA), and the wild-type synthase are shown. Panel A: 5 min products separated on a PolySep-4000 column. Panel B: 30 min products separated on a PolySep-6000 column. For comparison, the 580-kDa dextran standard eluted at 12.5 min or 16.8 min on the 4000 or 6000 column, respectively.

Specific changes in any of these residues can produce a modified hyaluronan or chondroitin that produces a hyaluronan or chondroitin product having a modified size distribution. Engineered changes to seHAS, spHAS, pmHAS, cvHAS, pmCS that decrease the intrinsic size of the hyaluronan or chondroitin polymer that the enzyme can make before the hyaluronan or chondroitin is released, will provide powerful means to produce either a hyaluronan or chondroitin polymer product of smaller or potentially larger size than the native enzyme.

Finally, larger molecular weight chondroitin made be degraded with specific chondroitinidases to make lower molecular weight chondroitin. This practice, however, is very difficult to achieve reproducibility and one must meticulously repurify the chondroitin to remove the chondroitinidases and unwanted digestion products.

Structurally modified chondroitin is no different conceptually than altering the size distribution of the chondroitin product by changing particular amino acids in the desired Chondroitin Synthase and/or more particularly, but not limiting thereto pmCS. Derivatives of UDP-GalNAc, in which the acetyl group is missing from the amide (UDP-GalN) or replaced with another chemically useful group (for example, phenyl to produce UDP-GalNPhe), are expected to be particularly useful. The free amino group would be available for chemical reactions to derivatize chondroitin in the former case with GAIN incorporation. In the latter case, GalNPhe, would make the polymer more hydrophobic or prone to making emulsions. The strong substrate specificity may rely on a particular subset of amino acids among the 10-20% that are conserved. Specific changes to one or more of these residues creates a functional chondroitin synthase that interacts less specifically with one or more of the substrates than the native enzyme. This altered enzyme could then utilize alternate natural or special sugar nucleotides to incorporate sugar derivatives designed to allow different chemistries to be employed for the following purposes: (i) covalently coupling specific drugs, proteins, or toxins to the structurally modified chondroitin for general or targeted drug delivery, radiological procedures, etc. (ii) covalently cross linking the hyaluronic acid itself or to other supports to achieve a gel, or other three dimensional biomaterial with stronger physical properties, and (iii) covalently linking hyaluronic acid to a surface to create a biocompatible film or monolayer.

### EXPERIMENTAL PROCEDURES

Materials and *Pasteurella* Strains - Unless otherwise noted, all chemicals were from Sigma or Fisher, and all molecular biology reagents were from Promega. The wild-type encapsulated Type F *P. multocida* strains, P-4679 and P-3695, were obtained from Dr. Richard Rimier (USDA, Ames, IA). These strains were isolated from turkeys with fowl cholera. P-4679 had a slightly larger capsule than P-3695 as judged by light microscopy and India Ink staining. The latter strain also possessed an endogenous uncharacterized plasmid.

Carbohydrate Analysis of Type F Capsular Material - The anionic polymer in the capsule of Type F bacteria was purified by urea extraction and cetylpyridinium chloride precipitation. P-4679 was grown in complete defined media (150 ml) with mild shaking overnight at 37°C. Cells were harvested by centrifugation (3,000 x g, 10 min) and washed twice with 0.1 M NaCl by repeated centrifugation and resuspension. The capsule was removed by extraction with 3 ml of 8 M urea for 8 min at 98°C. The cells were removed by high-speed centrifugation (15,000 x g, 10 min) and the urea solution was extracted with one volume of chloroform thrice at 22°C. GAGs in the aqueous extract were precipitated by the addition of cetylpyridinium chloride (1% w/v final concentration). Substantial polysaccharide may also be precipitated from the spent culture media in a similar fashion. After standing for 10 min, the precipitate was collected by high-speed centrifugation and redissolved in 2.5 M NaCl. The mixture was clarified by high-speed centrifugation and the supernatant was precipitated with 3 vol of ethanol. The precipitate was washed with 70% ethanol, dried slightly, and resuspended in 2.5 M NaCl. The ethanol precipitation procedure was repeated and the pellet was redissolved in water. Another round of ethanol precipitation (2 vol) was performed. The final GAG pellet was dissolved in water. The yield (0.6 mg uronic acid from all extract) was determined with the carbazole assay for uronic acid using a glucuronolactone standard.

The monosaccharide composition of the GAG extract was determined by acid hydrolysis (2 M HCl, 4 hrs, 100° C) and high pH anion exchange chromatography. The hydrolyzate was repeatedly diluted in water and dried under vacuum to remove HCl, then mixed with a rhamnose standard, and clarified using a 0.2 µm spin filter. Portions of the hydrolyzate (*about 5* nmoles of uronic acid) were injected onto a PA-I column (Dionex) equilibrated with 12 mM NaOH. After isocratic elution (25 min) to separate the neutral sugars, a gradient of sodium acetate (0 to 0.18 M in 30 min) was utilized to separate the acidic sugars. Eluted compounds were detected by pulsed amperometric detection. In parallel runs, the Type F sample was spiked with known monosaccharide standards of authentic chondroitin sulfate C (derived from shark cartilage) hydrolyzate. HA and heparin hydrolyzate standards were also run. Retention times relative to the rhamnose internal standard were calculated.

PCR Analysis of Type F Genomic DNA - Preliminary data from Southern blot analysis using pmHAS hybridization probes suggested that the Type A and the Type F microbes were very homologous at the capsule locus. PCR was utilized to verify these findings. Type F chromosomal DNA (0.1 µg) served as a template in PCR reactions (20 µl) utilizing oligonucleotide primers corresponding to various regions of the Type A capsule locus genes. After 40 cycles of PCR (94°C 30 5; 42°C 30 5; 72°C 4 min) with *Taq* DNA polymerase in the supplied buffer (Fisher), the samples were separated by agarose gel electrophoresis. Many primer pairs, but not all, amplified Type F DNA to yield products of the predicted size assuming that Type A and Type F loci were homologous. Two primers (Pm10, 5'-CACTGTCTAACTTTATTGTTAGCC-3' SEQ ID NO: 5; Pm*21,5'*-TTTTTAACGAATAGGCTGTC-3' SEQ ID NO:6) were chosen to amplify a 3.6 kb portion of the Type F locus predicted to contain the DNA encoding carboxyl-terminal half of the KfaA homolog and the amino-terminal portion of the putative polysaccharide synthase. The product from a PCR reaction (26 cycles) was cloned into a TA vector (Invitrogen) according to the manufacturer guidelines. The plasmid was analyzed by cycle sequencing (ThermoSequenase® system with ³³P-terminators, Amersham) with the Pm10 or the Pm21 primer. The preliminary sequence data from the PCR product derived from Type F DNA was highly (about 80%) homologous to the sequence of the Type A locus. Therefore, the 3.6-kb insert was excised from the plasmid, gel-purified, and labeled with digoxigenin (High Prime system, Boehringer Mannheim) to serve as a hybridization probe.

Isolation of Capsule Biosynthesis Locus DNA - A lambda library of Sau3A partially digested P-4679 DNA (4-9 kb average length insert) was made using the BamHI-cleaved )Zap ExpressTM vector system (Stratagene). The plaque lifts were screened by hybridization (5x SSC, 50°C; 16 hrs) with the digoxigenin-labeled probe using the manufacturer guidelines for colorimetric development. *E. coli* XLI-Blue MRF' was co-infected with the purified, individual positive lambda clones and ExAssist helper phage to yield phagemids. The resulting phagemids were transfected into *E. coli* XLOLR cells to recover the plasmids. Sequence analysis of the plasmids revealed a novel open reading frame, i.e. pmCS, with high homology to pmHAS (87%).

Expression of Recombinant *P. multocida* Chondroitin Synthase - In previous studies with pmHAS, a functional, soluble enzyme was created if a portion of the carboxyl terminus was truncated by molecular genetic means. Therefore, a portion of the pmCS ORF (residues 1-704) in the insert of one of the excised lambda clones, pPmF4A, was amplified by 20 cycles of PCR with *Taq* polymerase. The sense primer corresponded to the sequence at the deduced amino terminus of the ORF and the antisense primer encoded the new carboxyl terminus followed by an artificial stop codon. The resulting PCR product was purified and concentrated using GeneClean. This insert was cloned using the pETBlue-1 Acceptor system (Novagen) according to the manufacturer's instructions. The Taq-generated single A overhang is used to facilitate the cloning of the open reading frame downstream of the T7 promoter and the ribosome binding site of the vector. The ligated products were transformed into *E. coli* NovaBlue and plated on LB carbenicillin (50 µg/ml) and tetracycline (15 µg/ml) under conditions for blue/white screening.

White or light blue colonies were analyzed by restriction digestion. A clone containing a plasmid with the desired truncated ORF, pPm-CS¹⁻⁷⁰⁴, was transformed into *E. coli* Tuner, the T7 RNA polymerase-containing expression host, and maintained on LB media with carbenicillin and chloramphenicol (34 µg/ml) at 30°C. Log phase cultures were induced with 13-isopropylthiogalactoside (0.2 mM final) for 5 hrs. The cells were harvested by centrifugation, frozen, and extracted for 20 mm with a mild detergent (bPer II reagent, Pierce) at 7°C in the presence of a broad-range protease inhibitor cocktail. The cells were removed by centrifugation and the soluble extract was used as the source of Chondroitin Synthase enzyme for *in vitro* assays.

Western Blot Analysis of Recombinant *P. multocida* Chondroitin Synthase -A monospecific polyclonal antibody was generated against a synthetic peptide (acetyl-LDSDDYLEPDAVELCLKEF-amide SEQ ID NO: 7) corresponding to residues 526 to 544 of the pmHAS protein. The bPer extracts of various recombinant *E. coli* strains were heated at 42°C for 10 mm in sample buffer before loading. After electrophoresis, semi-dry transfer to a nitrocellulose membrane was performed. The Western blots were blocked with bovine serum albumin and incubated with the affinity-purified antibody before detection with a Protein A-alkaline phosphatase conjugate and colorimetric development with bromochloroindolyl phosphate and nitro blue tetrazolium.

Assays for Chondroitin Synthase Activity - Incorporation of radiolabeled monosaccharides from UDP-[¹⁴C]GlcUA and/or UDP-[³H]GalNAc precursors (NEN) was used to monitor chondroitin synthase activity. Samples were usually assayed in a buffer containing 50 mM Tris, pH 7.2, 20mM MnCl₂, 0.1 M (NH₄)₂SO₄, 1 M ethylene glycol, 0-0.6mM UDP-GlcUA, and 0-0.6 mM UDP-GalNAc in the presence of a chondroitin-6-sulfate acceptor oligosaccharide, [GalNAc-6-SO₄GlcUA-GalNAc-6-SO_{4]}n (*n* - 1 or 2; gift of Dr. Geetha Sugumaran), at 30°C. The reaction products were separated from substrates by descending paper (Whatman 3M) chromatography with ethanol/1M ammonium acetate, pH 5.5, development solvent (65:35). The origin of the paper strip was cut out, eluted with water, and the incorporation of radioactive sugars into HA polymer was detected by liquid scintillation counting with BioSafe II cocktail (RPI). To test the transfer specificity of pmCS¹⁻⁷⁰⁴ various UDP-sugars (UDP-GlcNAc, UDP-GalUA, UDP-Glc) were substituted for the authentic chondroitin precursors.

Size Analysis and Enzymatic Degradation of Labeled Polymers - Gel filtration chromatography was used to analyze the size distribution of the labeled polymers. Separations were performed with a Polysep-GFC-P 5000 column (300x7.8 mm; Phenomenex) eluted with 0.2 M sodium nitrate at 0.6 ml/min. Radioactivity was monitored with an in-line Radioflow LB508 detector (EG & G Berthold) using Unisafe I cocktail (1.8 ml/min; Zinsser). The column was standardized with fluorescein-labeled dextrans of various sizes. To identify the radiolabeled polymers, portions of some reactions were dialyzed into water (3 kDa cutoff) and the high molecular weight product was digested with various glycolytic enzymes for 7 hours at 37°C. The enzyme concentrations and digestion buffers were: *Flavobacterium* chondroitin AC lyase, 1 milliunit/µl, 50 mM Tris-acetate, pH 7.5; *Proteus* chondroitin AC lyase, 1 milliunit/µl, 50 mM Tris-acetate, pH 8; *Streptomyces* HA lyase, 266 milliunits/µl, 50 mM sodium acetate, pH 5.4.

If cells without formidable cell membrane barriers are used as host cells, transfection is carried out by the calcium phosphate precipitation method, well known to those of skill in the art. However, other methods may also be used for introducing DNA into cells such as by nuclear injection, cationic lipids, electroporation, protoplast fusion or by the Biolistic(tm) Bioparticle delivery system developed by DuPont (1989). The advantage of using the DuPont system is a high transformation efficiency. If prokaryotic cells or cells which contain substantial cell wall constructions are used, the preferred method of transfection is calcium treatment using calcium chloride to induce competence or electroporation.

Construction of suitable vectors containing the desired coding and control sequences employ standard ligation techniques. Isolated plasmids or DNA fragments are cleaved, tailored, and religated in the form desired to construct the plasmids required. Cleavage is performed by treating with restriction enzyme (or enzymes) in suitable buffer. In general, about 1 µg plasmid or DNA fragments are used with about 1 unit of enzyme in about 20 µl of buffer solution. Appropriate buffers and substrate amounts for particular restriction enzymes are specified by the manufacturer. Incubation times of about 1 hour at 37° C are workable.

After incubations, protein is removed by extraction with phenol and chloroform, and the nucleic acid is recovered from the aqueous fraction by precipitation with ethanol. If blunt ends are required, the preparation is treated for 15 minutes at 15° C with 10 units of Polymerase I (Klenow), phenolchloroform extracted, and ethanol precipitated. For ligation approximately equimolar amounts of the desired components, suitably end tailored to provide correct matching are treated with about 10 units T4 DNA ligase per 0.5 µg DNA. When cleaved vectors are used as components, it may be useful to prevent religation of the cleaved vector by pretreatment with bacterial alkaline phosphatase.

For analysis to confirm functional sequences in plasmids constructed, the first step was to amplify the plasmid DNA by cloning into specifically competent *E. coli* SURE cells (Stratagene) by doing transformation at 30-32°C. Second, the recombinant plasmid is used to transform *E. coli* K5 strain Bi8337-41, which can produce the UDP-GlcA precursor, and successful transformants selected by antibiotic resistance as appropriate. Plasmids from the library of transformants are then screened for bacterial colonies that exhibit HA production. These colonies are picked, amplified and the plasmids purified and analyzed by restriction mapping. The plasmids showing indications of a functional Chondroitin Synthase gene are then further characterized by any number of sequence analysis techniques which are known by those of ordinary skill in the art.

In general, prokaryotes are used for the initial cloning of DNA sequences and construction of the vectors useful in the invention. It is believed that a suitable source may be bacterial cells, particularly those derived from strains that can exist on a simple minimal media for ease of purification. Bacteria with a single membrane, but a thick cell wall such as *Staphylococci* and *Streptococci* are Gram-positive. Gram-negative bacteria such as *E. coli* contain two discrete membranes rather than one surrounding the cell. Gram-negative organisms tend to have thinner cell walls. The single membrane of the Gram-positive organisms is analogous to the inner plasma membrane of Gram-negative bacteria. Additionally, many bacteria possess transport systems that help capsular polymers be secreted from the cell.

For the expression of Chondroitin Synthase in a form most likely to accommodate Chondroitin Synthase synthesis, one may desire to employ *Streptococcus* species such as *S. equisimilis* or *S. zooepidemicus and*/*or P. multocida and*/*or Bacillus Strains.* The aforementioned strains, as well as *E. coli* W3110 (F-, lambda-, prototrophic, ATCC No. 273325), bacilli such as *Bacillus subtilis,* or other enterobacteriaceae such as *Serratia marcescens,* could be utilized to generate a "super" Chondroitin Synthase containing host.

In general, plasmid vectors containing origins of replication and control sequences which are derived from species compatible with the host cell are used in connection with these hosts. The vector ordinarily carries an origin of replication, as well as marking sequences which are capable of providing phenotypic selection in transformed cells. For example, *E. coli* is typically transformed using pBR322, a plasmid derived from an *E. coli* species. pBR322 contains genes for ampicillin and tetracycline resistance and thus provides easy means for identifying transformed cells. A pBR plasmid or a pUC plasmid, or other microbial plasmid or phage must also contain, or be modified to contain, promoters which can be used by the microbial organism for expression of its own proteins.

Those promoters most commonly used in recombinant DNA construction include the *lacZ* promoter, *tac* promoter, the T7 bacteriophage promoter, and tryptophan (trp) promoter system. While these are the most commonly used, other microbial promoters have been discovered and utilized, and details concerning their nucleotide sequences have been published, enabling a skilled worker to ligate them functionally with plasmid vectors. Also for use with the present invention one may utilize integration vectors.

In addition to prokaryotes, eukaryotic microbes, such as yeast cultures may also be used. *Saccharomyces cerevisiae,* or common baker's yeast is the most commonly used among eukaryotic microorganisms, although a number of other strains are commonly available. For expression in *Saccharomyces,* the plasmid YRp7, for example, is commonly used. This plasmid already contains the *trp1* gene which provides a selection marker for a mutant strain of yeast lacking the ability to grow without tryptophan, for example, ATCC No. 44076 or PEP4-1. The presence of the *trp1* lesion as a characteristic of the yeast host cell genome then provides an effective environment for detecting transformation by growth in the absence of tryptophan. Suitable promoting sequences in yeast vectors include the promoters for the galactose utilization genes, the 3-phosphoglycerate kinase or other glycolytic enzymes, such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

In constructing suitable expression plasmids, the termination sequences associated with these genes are also ligated into the expression vector 3' of the sequence desired to be expressed to provide polyadenylation of the mRNA and termination. Other promoters, which have the additional advantage of transcription controlled by growth conditions are the promoter region for alcohol dehydrogenase 2, cytochrome C, acid phosphatase, degradative enzymes associated with nitrogen metabolism, and the aforementioned glyceraldehyde-3-phosphate dehydrogenase, and enzymes responsible for maltose and galactose utilization. Any plasmid vector containing a yeast-compatible promoter, origin of replication and termination sequences is suitable.

In addition to microorganisms, cultures of cells derived from multicellular organisms may also be used as hosts. In principle, any such cell culture is workable, whether from vertebrate or invertebrate cultures. However, interest has been greatest in vertebrate cells, and propagation of vertebrate cells in culture has become a routine procedure in recent years. Examples of such useful host cell lines are VERO and HeLa cells, Chinese hamster ovary (CHO) cell lines, and WI38, BHK, COS, and MDCK cell lines.

For use in mammalian cells, the control functions on the expression vectors are often provided by viral material. For example, commonly used promoters are derived from polyoma, Adenovirus 2, bovine papilloma virus and most frequently Simian Virus 40 (SV40). The early and late promoters of SV40 virus are particularly useful because both are obtained easily from the virus as a fragment which also contains the SV40 viral origin of replication. Smaller or larger SV40 fragments may also be used, provided there is included the approximately 250 bp sequence extending from the *Hind III* site toward the *Bg1 I* site located in,the viral origin of replication.

Further, it is also possible, and often desirable, to utilize promoter or control sequences normally associated with the desired gene sequence, provided such control sequences are compatible with the host cell systems. An origin of replication may be provided either by construction of the vector to include an exogenous origin, such as may be derived from SV40 or other viral (e.g., Polyoma, Adeno, BPV) source, or may be provided by the host cell chromosomal replication mechanism. If the vector is integrated into the host cell chromosome, the latter mechanism is often sufficient.

Chondroitin sulfate and dermatan sulfate are both derived from the same polymer, i.e. D-glucuronic acid beta (1-3)D-N-acetyl galactosamine beta (1-4). Both chondroitin sulfate and dermatan sulfate can be sulfated at positions 4 or 6 of N-acetyl galactosamine and position 2 of the uronic acid. Neither has been observed to be N-sulfated in nature. The difference between chondroitin sulfate and dermatan sulfate is the epimerisation of glucuronic acid to iduronic acid. There are problems however with the nomenclature and designation of a polysaccharide as either chondroitin sulfate or dermatan sulfate. In particular, the frequency with which iduronic acid must occur rather than glucuronic acid, for the chain to be called a dermatan sulfate chain, is open to interpretation.
Thus a chondroitin sulfate chain may have sequences of dermatan sulfate interspersed therein and visa versa. One of ordinary skill in the art would appreciate, however, that a polymer having between 10% and 50% epimerisation of glucuronic acid to iduronic acid would be suitably designated a dermatan sulfate polysaccharide.

A chondroitin polymer is produced by a chondroitin synthase and in particular, but not limited thereto, the pmCS encoded by the purified acid segment of the present invention. For example, the chondroitin polymer can be converted into a dermatan molecule that may be an even more valuable product than chondroitin itself. The chondroitin polymer can be converted into dermatan either in the purified form or *in vivo* (i.e. in the host itself). For example, Chang et al. have identified and detailed a reaction of *Azotobacter vinelandii* poly-beta-D-mannuronic acid C-5-epimerase on synthetic D-glucuronans. A dermatan molecule can be made using the *Azotobacter vinelandii* poly-beta-(1->4)-D-mannuronic acid C-5-epimerase to react with a chondroitin polymer made via a chondroitin synthase such as pmCS. (Chang et al. Action of Azotobacter vinelandii poly-beta-D-mannuronic Acid C-5-epimerase on Synthetic D-Glucuronans, Carbohydrate Research, Dex. 1,2000; 329(4):913-22).

U.S. Patent No. 5,939,289 issued to Ertesvag et al., also discloses a C-5 epimerase which may be used to convert the chondroitin molecule produced by the *P. multocida* chondroitin synthase into a unsulfated dermatan molecule. The c-5 epimerase is expected to work on the chondroitin polymer as the Chang et al. paper describes epimerization of a variety of polysaccharides containing uronic acids including oxidized starch and chitin.

Alternatively, instead of step-wise chondroitin synthesis followed by epimerization reaction, an *in vivo* combined method should be possible. This is very suitable in pmCS/*Azotobacter* epimerase case as the reactions are compatible and both genes are from Gram- negative bacteria. Both enzymes have been shown to function in *E-coli.* Placing both genes in one cell and allowing contact of chondroitin and the epimerase results in the desired product.

Further, an assay procedure for measuring the reactions catalyzed by polyuronic acid, C-5 epimerases can be used. (See e.g., Chang et al. Measurement of the Activity of Polyuronic Acid C-5 Epimerases, Anal. Biochem., April 10, 1998; 258(1):59-62).

Action of C-5 epimerases inverts the C-6 carboxyl group of polyuronic acids thus converting beta-linked residues into alpha-linked residues or vice versa. The above-identified assay takes advantage of the greater susceptibility of the acid hydrolysis of alpha-glycosidic linkages than beta-glycosidic linkages. Thus, acid treatment of experimental polymers (the product) results in a color yield but the parent starting material does not result in a substantial color yield. The method of this particular assay involves the partial acid hydrolysis of the polyuronic acid before and after reaction witch the C-5 epimerase. The greater or lesser amounts of uronic acid released (solubilized) before and after reaction of the C-5 epimerase are a measure of the amount of alpha- or beta-glycosidic linkages that are formed and a measure of the amount of catalysis by the enzyme. In this manner, the conversion chondroitin polymer to dermatan can be catalyzed and monitored for reaction and efficiency.

The chondroitin molecule made by the pmCS enzyme is an ideal polymeric starting material for the creation of a dermatan sulfate molecule. Certain mammalian epimerases only epimerize unsulfated polymer molecules. For example, the C-5 uronosyl epimerase, which is capable of converting a chondroitin molecule into a dermatan molecule, will only epimerize an unsulfated chondroitin molecule. Unsulfated chondroitin molecules are not found in nature, and chondroitin sulfate must be either desulfated or an unsulfated chondroitin molecule must be recombinantly produced. Since no chondroitin synthase has been known prior to or since the discovery of the pmCS enzyme, one of ordinary skill in the art would have to expend additional time, money, and capital in order to convert sulfated chondroitin into unsulfated or desulfated chondroitin. Once the chondroitin is unsulfated, the mammalian epimerases can be used to convert the chondroitin molecule into a dermatan molecule. By utilizing a chondroitin synthase, such as pmCS, one of ordinary skill in the art is capable of producing an unsulfated chondroitin molecule which is an ideal starting material for epimerization by a mammalian epimerase. For one such mammalian epimerase and methods of using same, see e.g. Malmstrom A., Biosynthesis of Dermatan Sulfate - Substrate Specificity of the C-5 Uronosyl Epimerase, J. Biol. Chem., Jan.10, 1984; 259(1): 161-5.

Utilizing enzymatic sulfation, the chondroitin polymer - turned - dermatan molecule can be sulfated, thereby creating an even more valuable and flexible polymer for anticoagulation, device coatings, and/or other biomaterial. As pointed out in the Eklund et al. article entitled Dermatan is a Better Substrate for 4-0-sulfation than Chondroitin: Implications in the Generation of 4-O-sulfated, L-iduronic-rich Galactosaminoglycans, Arch. Biochem. Biophys., Nov. 15, 2000; 383(2):1771-7, dermatan is not only more easily enzymatically sulfated than chondroitin, but sulfated dermatan is a more valuable, flexible and useful product than chondroitin. Thus, utilizing a chondroitin synthase such as pmCS, one or ordinary skill in the art, given the present disclosure, would be able to produce natural and non-natural chondroitin as well as dermatan and dermatan sulfate. Other articles outline other methodologies for enzymatically sulfating dermatan: Bhakta et al. Sulfation of N-acetylglucosamine by Chondroitin 6-Sulfotransferase 2 (GST-5), J.Biol. Chem., Dec. 22, 2000; 275(51):40226-34; Ito et al. Purification and Characterization of N-acetylgalactosamine 4-sulfate 6-O-Sulfotransferase from the Squid Cartilage, J. Biol. Chem., Nov. 3, 2000; 275(44): 34728-36.

In addition to enzymatic sulfation, the dermatan polymer can be chemically sulfated. One method for chemical sulfation is outlined in the article by Garg et al. entitled Effect of Fully Sulfated Glycosaminoglycans on Pulmonary Artery Smooth Muscle Cell Proliferation, Arch. Biochem. Biophys., Nov. 15, 1999; 371(2): 228-33.

Typically, the polysaccharide in an anhydrous solvent is treated with sulfur trioxide or chlorosulfonic acid. In any event, one of ordinary skill in the art given the chondroitin synthase (pmCS) encoded by the purified nucleic acid segment of the present invention and the methodology for producing a chondroitin polymer from the pmCS enzyme, would be capable of using the epimerization reaction to form a dermatan molecule and then sulfating this dermatan molecule by known enzymatic, or chemical sulfation techniques. Alternatively, unepimerimized chondroitin could be sulfated by any means as well.

Also, U.S. Patent No. 4,990,601 issued to Skjak-Braek et al., discloses a chemical process using supercritical CO₂ which epimerizes uronic acid in a compound. Utilizing a chondroitin polypeptide using the pmCS encoded by the purified nucleic acid segment of the present invention and the CO₂ epimerization method of Skjak-Braek et al., one of ordinary skill in the art can easily make unsulfated dermatan molecules.

Compositional Analysis of Type F *P. multocida* Polymer - Previous work by others had shown that the Type F capsule is removed from bacterial cells by treatment with chondroitin AC lyase. We found that a fragment of the specific HA-binding protein, aggrecan, in the HA-TEST assay (Pharmacia) did not cross-react with extracts of the Type F polymer, but readily detected the HA in parallel extracts from Type A bacteria. Acid hydrolysis and monosaccharides analysts of the Type F polymer showed that it contained the sugars galactosamine and GlcUA (Table 2 and Fig. 5).

**TABLE 2**

| | | | | | |
|---|---|---|---|---|---|
| Monosaccharide Composition of Type F Polymer and Various GAGs. Acid hydrolysis and high pH ion exchange chromatography were utilized to determine the sugars components of the Type F polymer **(F).** The polysaccharides chondroitin sulfate C **(C),** hyaluronan **(HA),** and heparin **(HEP),** and pure monosaccharides were used as standards. Under these hydrolysis conditions, deacetylation and desulfation as well as the desired fragmentation of glycosidic bonds occur. Retention times relative to the internal standard rhamnose elution time (10.7 min; set to 1) are presented for the relevant hexosamines. Acidic sugars were eluted with a sodium acetate gradient; the retention time of the major uronic acid peak from the start of the gradiant is presented. Type F polysaccharide and chondroitin sulfate possess the identical monsaccharide composition, galactosamine and glucuronic acid. | | | | | |

| **Polysaccharides** | | | | | |
|---|---|---|---|---|---|
| **Sugar** | **C** | **C/F MIX** | **F** | **HA** | **HEP** |
| | **Retention Time Relative to Rhamnose** | | | | |
| glucosamine | ND* | ND | ND | 1.38 | 1.38 |
| galactosamine | 1.14 | 1.12 | 1.12 | ND | ND |

| | **Retention time (min)** | | | | |
|---|---|---|---|---|---|
| uronic acid | 14.87 | 14.87 | 14.87 | 14.85 | 14.58 |

| | | | | | |
|---|---|---|---|---|---|
| * ND, not detected | | | | | |

The ion exchange profile of the chondroitin sulfate hydrolyzate was indistinguishable from the Type F hydrolyzate; mixing experiments demonstrated that the component peaks migrated identically. No other sugars were detected in the Type F polymer including glucosamine, mannose, galactose, glucose, and fucose. Hydrolyzates of the HA and heparin standards clearly contained glucosamine but not galactosamine. Preliminary NMR studies are consistent with the hypothesis that the amino sugar of the Type F polymer is present in an acetylated form (NAc CH₃ chemical shift at 2.02 ppm in D₂0; University of Georgia Complex Carbohydrate Research Center) (Fig. 8). Disaccharide analysis of the chondroitin from Type F is of the correct mass and charge expected to be derived from unsulfated chondroitin (Figs. 9 and 10). This process involves cleaving the polymer with chondroitinase, separating products by capillary electrophoresis. The retention time is compared to authentic standards. Mass was measured by mass spectrometry; in this size range, exact masses to within 1 Da are measured.

Molecular Cloning of the Type F *P. multocida* Capsular Locus PCR products were obtained utilizing Type F chromosomal DNA as a template and various oligonucleotide primers corresponding to the Type A capsule locus. A 3.6 kb PCR product, which contained large portions of the Type F *KfaA* homolog (a putative polysaccharide transporter of *E. coli*) and the putative pmCS gene, was used as a hybridization probe to obtain an intact *P. multocida* capsular locus from a lambda library. Two positively hybridizing plaques were found after screening about 10⁴ plaques, and these phage were converted into plasmids. We found that both plasmids contained a novel open reading frame of 965 residues, which we named pmCS, that was highly homologous to the Type A HA synthase, pmHAS (Fig. 1). The level of identity was about 87 % at both the DNA and protein levels. The differences in amino acid sequence were mainly localized to several regions of the polypeptide in the amino terminal half of the molecules. There is an excellent overall alignment of the enzymes except for a 7-residue insertion in the pmHAS sequence in the position corresponding to residue 53 of the pmCS sequence.

The central portion of both the pmCS and the pmHAS polypeptides (residues 430-530) is most homologous to bacterial glycosyltransferases from a wide variety of genera, including *Streptococcus, Vibric, Neisseria* and *Staphylococcus,* that form exopolysaccharides or the carbohydrate portions of lipopolysaccharides. The some of the most notable sequence similarities are the DGSTD and the DxDD motifs. Directly downstream of the *pmCS* gene a putative UDP-glucose dehydrogenase gene was identified. Therefore, the relative gene order *[KfaA* homolog - polysaccharide synthase gene - UDP-glucose dehydrogenase gene] in this portion of the *Pasteurella* Type F capsule operon is the same as that found in Type A.

Heterologous Expression of a Functional *P. multocida* Chondroitin Synthase - Western blot analysis using a monospecific antipeptide antibody was used to detect the production of pmCS¹⁻⁷⁰⁴ or pmHAS¹⁻⁷⁰³ polypeptide (Fig. 2). Both enzymes contain a sequence that corresponds exactly to the synthetic peptide used to generate the antibody. Extracts derived from *E. coli* Tuner cells containing the pmCS¹⁻⁷⁰⁴plasmid contained an immunoreactive band of the appropriate size (*i.e.* predicted to be 80 kDa), but this band was not present in samples from cells with the vector alone control. The use of soluble pmCS¹⁻⁷⁰⁴ protein provided increased expression levels and facilitated preparation of enzyme in comparison to use of the native-length membrane protein.

Extracts derived from *E. coli* Tuner cells containing the pmCS¹⁻⁷⁰⁴ plasmid, but not samples from cells with the vector alone, synthesized polymer *in vitro* when supplied with both UDP-GlcUA and UDP-GalNAc simultaneously (Table 3).

**TABLE 3**

| | | |
|---|---|---|
| Transferase Specificity of Recombinant pmCS¹⁻⁷⁰⁴ for Sugar Nucleotides. Crude bPer extract (150 µg of total protein) was incubated in 50 µl of assay buffer containing 0.5 µg of chondroitin oligosaccharide acceptor for 20 min either with UDP-[¹⁴C]GlcUA or UDP-[³H]GalNAc. The radiolabeled sugar (300 µM, 0.04 µCi) was used in the presence of the indicated second unlabeled sugar nucleotide (600 µM). The incorporation into polymer was assessed by paper chromatography. The relative percentage of incorporation in comparison to the assay containing the authentic precursor (set to 100%) is shown in *parentheses.* A representative experiment is shown. The recombinant pmCS¹⁻⁷⁰⁴ incorporated only the authentic chondroitin precursors into polysaccharide. | | |

| | **Incorporation of first sugar** | |
|---|---|---|
| **Second sugar nucleotide Present** | **[¹⁴C]GlcUA** | **[³H]GalNAc** |
| | **dpm (%)** | |
| None | 60 (0.9) | 250 (7.5) |
| UDP-GlcUA | ND* | 3,310 (100) |
| UDP-GalUA | ND | 315 (9.5) |
| UDP-GalNAc | 6,590 (100) | ND |
| UDP-GlcNAc | 85 (1.5) | ND |
| UDP-Glc | 60 (0.9) | 370(11) |

| | | |
|---|---|---|
| *ND, not determined. | | |

No incorporation of radiolabeled [¹⁴C]GlcUA into polymer was observed if UDP-GalNAc was omitted, or if UDP-GlcNAc was substituted for UDP-GalNAc. Conversely, in experiments using UDP-[³H]GalNAc, substantial incorporation of radiolabel into polymer was only noted when UDP-GlcUA was also present. UDP-GalUA or UDP-Glc did not substitute for UDP-GlcUA. No polymerization or transferase activity was detected if the divalent metal ions were chelated with EDTA. The addition of the chondroitin oligosaccharide acceptor increased sugar incorporation catalyzed by pmCS¹⁻⁷⁰⁴ at least 50- to 100-fold in comparison to parallel reactions without acceptor in analogy to observations of pmHAS.

Analysis by gel filtration chromatography indicated that recombinant pmCS produced polymer chains of 10³ = (1,000) monosaccharides long (100 to 400 kDa) *in vitro.* Radioactivity from both labeled GlcUA and GalNAc sugars co-migrated as a single peak (Fig. 3A). No radiolabel was incorporated into high molecular weight polymer if both UDP-sugars were not present during the assay. The identity of the polymer as chondroitin was verified by its sensitivity to *Flavobacterium* or *Proteus* chondroitin AC lyase (Fig. 3B) and its resistance to the action of *Streptomyces* HA lyase (Fig. 3C).

Analysis of the native Type F polymer by agarose gel electrophoresis also shows that high molecular weight polymer is made by bacteria *in vivo* (Fig. 4). The Type F polymer was visualized by stains. All staining an 0.8% agarose gel run in 1x TAE system. HA and Type F both stained blue while DNA and chondroitin sulfate stained purple and Heparin stained yellow. The Type F polymer is smaller than HA, but still forms very large chains of about 50 to about 150 KDa.

*P. multocida* Type F produces a chondroitin capsule. The glycosyltransferase responsible for polymerizing the chondroitin backbone component of the capsular polysaccharide has also been cloned. The pmCS enzyme appears to be a close homolog of the pmHAS enzyme. Recently, it was determined that the pmHAS enzyme contains two active sites in a single polypeptide by generating mutants that transfer only GlcUA or only GlcNAc. Mixing the two different mutant proteins reconstituted the HA synthase activity. It is likely that one domain, called A1, is responsible for GlcNAc transfer and the other domain, called A2, is responsible for GlcUA transfer. Comparison of the pmHAS and the pmCS sequences reveals that the majority of the sequence differences exist in the A1 domain. The pmCS enzyme transfers a different hexosamine, GalNAc, thus being consistent with the proposed two-domain structure for pmHAS.

The pmHAS protein was also hypothesized to interact with a putative polysaccharide transporter system or a membrane-bound partner via its carboxyl terminus because deletion of residues 704 to 972 from the native-length enzyme resulted in the formation of a soluble enzyme. However, no substantial membrane-associated or hydrophobic regions are predicted to reside in this sequence. As pmHAS and pmCS are highly homologous in this region, which is not essential for their glycosyltransferase activities, it is quite likely that the carboxyl terminus contains domains or motifs required for interacting with the polysaccharide transport machinery or a membrane-bound partner *in vivo.* The evolutionary relationship between Type A and Type F *P. multocida* strains has not yet been delineated. Both organisms are widespread causative agents of fowl cholera, but many more isolates from diseased birds in North America are Type A microbes with HA capsules. It is likely that the progenitor of the two distinct capsular types had either a chondroitin synthase-like or a HAS-like gene. The specificity of this ancestral enzyme may have changed after a few mutations resulting in the appearance of another capsular type. Apparently, the sugar transfer specificity is rather selective since neither recombinant pmCS nor pmHAS misincorporate the inappropriate hexosamine into polymer *in vitro.* Some Gram-negative bacteria (*e.g. E. coli*) possess an UDP-GlcNAc/UDP-GalNAc epimerase, therefore the hexosamine precursor either for HA or for chondroitin could have been available for polysaccharide biosynthesis without the need to gain an auxiliary metabolic enzyme simultaneously. Typically the UDP-glucose dehydrogenase, the enzyme that forms the UDP-GlcUA precursor, is found in Gram-negative bacteria only if the microbe possesses a GlcUA-containing polymer or glycoconjugate. In both Type A and Type F *P. multocida,* the UDP-glucose dehydrogenase gene is directly downstream of the GAG synthase.

The relationship between the bacterial chondroitin synthase and the putative mammalian counterpart is unclear. No similar vertebrate proteins are deposited in the database as yet. Both bacterial pmCS and the vertebrate chondroitin synthase utilize UDP-sugars to extend acceptor carbohydrates *in vitro.* In most cases, the mammalian enzyme in cell-free extracts, however, does not produce long chondroitin chains and only the half-reaction (*e.g.* adding a single GlcUA to a GalNAc-terminated oligosaccharide or *vice versa*) is readily observed *in vitro.* In vertebrate tissues, other enzymes modify chondroitin extensively by sulfation and/or epimerization. The discovery and the characterization of pmCS will assist the further study of the rather recalcitrant mammalian chondroitin synthase enzymes.

Thus, it should be apparent that a purified nucleic acid segment having a coding region encoding enzymatically active chondroitin synthase has been provided in accordance with appended claim 1, methods of producing chondroitin from the pmCS gene, and the use of chondroitin produced from a chondroitin synthase encoded by the pmCS gene, that fully satisfies the objectives and advantages set forth above. Although the invention has been described in conjunction with specific embodiments thereof, it is evident that many alternatives, modifications, and variations will be apparent to those skilled in the art. Accordingly, it is intended to embrace all such alternative, modifications, and variations that fall within the scope of the appended claims.

All of the numerical and quantitative measurements set forth in this application (including in the examples and in the claims) are approximations.

The invention illustratively disclosed or claimed-herein suitably may be practiced in the absence of any element which is not specifically disclosed or claimed herein. Thus, the invention may comprise, consist of, or consist essentially of the elements claimed herein.

The following claims are entitled to the broadest possible scope consistent with this application. The claims shall not necessarily be limited to the preferred embodiments or to the embodiments shown in the examples.
<110> Paul DeAngelis
<120> CHONDROITIN SYNTHASE GENE AND METHODS OF MAKING AND USING SAME
<130> 5820.601
<140> Not Yet Assigned
<141> Herewith
<160> 7
<170> WordPerfect 8.0 (saved in ASCII format)
<210> 1
<211> 2979 base pairs
<212> DNA
<213> Pasteurella Multocida
<220>
<400> pmCS DNS Sequence 
1 ttataaactg attaaagaag gtaaacgatt caagcaaggt taatttttaa aggaaagaaa
61 atgaatacat tatcacaagc aataaaagca tataacagca atgactatga attagcactc
121 aaattatttg agaagtctgc tgaaacctac gggcgaaaaa tcgttgaatt ccaaattatc
181 aaatgtaaag aaaaactctc gaccaattct tatgtaagtg aagataaaaa aaacagtgtt
241 tgcgatagct cattagatat cgcaacacag ctcttacttt ccaacgtaaa aaaattaact
301 ctatccgaat cagaaaaaaa cagtttaaaa aataaatgga aatctatcac tgggaaaaaa
361 tcggagaacg cagaaatcag aaaggtggaa ctagtaccca aagattttcc taaagatctt
421 gttcttgctc cattgccaga tcatgttaat gattttacat ggtacaaaaa tcgaaaaaaa
481 agcttaggta taaagcctgt aaataagaat atcggtcttt ctattattat tcctacattt
541 aatcgtagcc gtattttaga tataacgtta gcctgtttgg tcaatcagaa aacaaactac
601 ccatttgaag tcgttgttgc agatgatggt agtaaggaaa acttacttac cattgtgcaa
661 aaatacgaac aaaaacttga cataaagtat gtaagacaaa aagattatgg atatcaattg
721 tgtgcagtca gaaacttagg tttacgtaca gcaaagtatg attttgtctc gattctagac
781 tgcgatatgg caccacaaca attatgggtt cattcttatc ttacagaact attagaagac
841 aatgatattg ttttaattgg acctagaaaa tatgtggata ctcataatat taccgcagaa
901 caattcctta acgatccata tttaatagaa tcactacctg aaaccgctac aaataacaat
961 ccttcgatta catcaaaagg aaatatatcg ttggattgga gattagaaca tttcaaaaaa
1021 accgataatc tacgtctatg tgattctccg tttcgttatt ttgttgcggg taatgttgca
1081 ttttctaaag aatggctaaa taaagtaggt tggttcgatg aagaatttaa tcattggggg
1141 ggcgaagatg tagaatttgg ttacagatta tttgccaaag gctgtttttt cagagtaatt
1201 gacggcggaa tggccatcca tcaagaacca cctggtaaag aaaatgaaac agaacgcgaa
1261 gctggtaaaa gtattacgct taaaattgtg aaagaaaagg taccttacat ctatagaaag
1321 cttttaccaa tagaagattc acatattcat agaatacctt tagtttctat ttatatcccc
1381 gcttataact gtgcaaatta tattcaaaga tgtgtagata gtgctcttaa tcaaactgtt
1441 gtcgatctcg aggtttgtat ttgtaacgat ggttcaacag ataatacctt agaagtgatc
1501 aataagcttt atggtaataa tcctagggta cgcatcatgt ctaaaccaaa tggcggaata
1561 gcctcagcat caaatgcagc cgtttctttt gctaaaggtt attacattgg gcagttagat
1621 tcagatgatt atcttgagcc tgatgcagtt gaactgtgtt taaaagaatt tttaaaagat
1681 aaaacgctag cttgtgttta taccactaat agaaacgtca atccggatgg tagcttaatc
1741 gctaatggtt acaattggcc agaattttca cgagaaaaac tcacaacggc tatgattgct
1801 caccatttta gaatgtttac gattagagct tggcatttaa cggatggatt taacgaaaat
1861 attgaaaacg ccgtggatta tgacatgttc cttaaactca gtgaagttgg aaaatttaaa
1921 catcttaata aaatctgcta taaccgcgta ttacatggtg ataacacatc cattaagaaa
1981 ctcggcattc aaaagaaaaa ccattttgtt gtagtcaatc agtcattaaa tagacaaggc
2041 atcaattatt ataattatga caaatttgat gatttagatg aaagtagaaa gtatatcttc
2101 aataaaaccg ctgaatatca agaagaaatg gatatgttaa aagatcttaa actcattcaa
2161 aataaagatg ccaaaatcgc agtcagtatt ttctatccca atacattaaa cggcttagtg
2221 aaaaaactaa acaatattat tgaatataat aaaaatatat tcgttattat tctacatgtt
2281 gataagaatc atcttacacc agacatcaaa aaagaaatat tggctttcta tcataagcac
2341 caagtgaata ttttactaaa taatgacatc tcatattaca cgagtaatag actaataaaa
2401 actgaggcac atttaagtaa tattaataaa ttaagtcagt taaatctaaa ttgtgaatac
2461 atcatttttg ataatcatga cagcctattc gttaaaaatg acagctatgc ttatatgaaa
2521 aaatatgatg tcggcatgaa tttctcagca ttaacacatg attggatcga gaaaatcaat
2581 gcgcatccac catttaaaaa gctgattaaa acctatttta atgacaatga cttaagaagt
 
2641 atgaatgtga aaggggcatc acaaggtatg tttatgaagt atgcgctacc gcatgagctt
2701 ctgacgatta ttaaagaagt catcacatcc tgccaatcaa ttgatagtgt gccagaatat
2761 aacactgagg atatttggtt ccaatttgca cttttaatct tagaaaagaa aaccggccat
2821 gtatttaata aaacatcgac cctgacttat atgccttggg aacgaaaatt acaatggaca
2881 aatgaacaaa ttcaaagtgc aaaaaaaggc gaaaatatcc ccgttaacaa gttcattatt
2941 aatagtataa cgctataaaa catttgcatt ttattaaaa
<110> Paul DeAngelis
<120> CHONDROITIN SYNTHASE GENE AND METHODS OF MAKING AND USING SAME
<130> 5820.601
<140> Not Yet Assigned
<141> Herewith
<160> 7
<170> WordPerfect 8.0 *Software*
<210> 2
<211> 965 amino acids
<212> PRT
<213> Pasteurella Multocida
<220>
<400> pmCS Protein Sequence 
1 mntlsqaika ynsndyelal klfeksaety grkivefqii kckeklstns yvsedkknsv
61 cdssldiatq lllsnvkklt lseseknslk nkwksitgkk senaeirkve lvpkdfpkdl
121 vlaplpdhvn dftwyknrkk slgikpvnkn iglsiiiptf nrsrilditl aclvnqktny
181 pfevvvaddg skenlltivq kyeqkldiky vrqkdygyql cavrnlglrt akydfvsild
241 cdmapqqlwv hsyltelled ndivligprk yvdthnitae qflndpylie slpetatnnn
301 psitskgnis ldwrlehfkk tdnlrlcdsp fryfvagnva fskewlnkvg wfdeefnhwg
361 gedvefgyrl fakgcffrvi dggmaihqep pgkenetere agksitlkiv kekvpyiyrk
421 llpiedshih riplvsiyip ayncanyiqr cvdsalnqtv vdlevcicnd gstdntlevi
481 nklygnnprv rimskpnggi asasnaavsf akgyyigqld sddylepdav elclkeflkd
541 ktlacvyttn rnvnpdgsli angynwpefs reklttamia hhfrmftira whltdgfnen
601 ienavdydmf lklsevgkfk hlnkicynrv lhgdntsikk lgiqkknhfv vvnqslnrqg
661 inyynydkfd dldesrkyif nktaeyqeem dmlkdlkliq nkdakiavsi fypntlnglv
721 kklnniieyn knifviilhv dknhltpdik keilafyhkh qvnillnndi syytsnrlik
781 teahlsnink lsqlnlncey iifdnhdslf vkndsyaymk kydvgmnfsa lthdwiekin
841 ahppfkklik tyfndndlrs mnvkgasqgm fmkyalphel ltiikevits cqsidsvpey
901 ntediwfqfa llilekktgh vfnktstlty mpwerklqwt neqiqsakkg enipvnkfii
961 nsitl
<110> Paul DeAngelis
<120> CHONDROITIN SYNTHASE GENE AND METHODS OF MAKING AND USING SAME
<130> 5820.601
<140> Not Yet Assigned
<141> Herewith
<160> 7
<170> WordPerfect 8.0 *Software*
<210> 3
<211> 2979 base pairs
<212> DNA
<213> Pasteurella Multocida
<220>
<400> pmCS DNA Sequence 
1 ttataaactg attaaagaag gtaaacgatt caagcaaggt taatttttaa aggaaagaaa
61 atgaatacat tatcacaagc aataaaagca tataacagca atgactatga attagcactc
121 aaattatttg agaagtctgc tgaaacctac gggcgaaaaa tcgttgaatt ccaaattatc
181 aaatgtaaag aaaaactctc gaccaattct tatgtaagtg aagataaaaa aaacagtgtt
241 tgcgatagct cattagatat cgcaacacag ctcttacttt ccaacgtaaa aaaattaact
301 ctatccgaat cagaaaaaaa cagtttaaaa aataaatgga aatctatcac tgggaaaaaa
361 tcggagaacg cagaaatcag aaaggtggaa ctagtaccca aagattttcc taaagatctt
421 gttcttgctc cattgccaga tcatgttaat gattttacat ggtacaaaaa tcgaaaaaaa
481 agcttaggta taaagcctgt aaataagaat atcggtcttt ctattattat tcctacattt
541 aatcgtagcc gtattttaga tataacgtta gcctgtttgg tcaatcagaa aacaaactac
601 ccatttgaag tcgttgttgc agatgatggt agtaaggaaa acttacttac cattgtgcaa
661 aaatacgaac aaaaacttga cataaagtat gtaagacaaa aagattatgg atatcaattg
721 tgtgcagtca gaaacttagg tttacgtaca gcaaagtatg attttgtctc gattctagac
781 tgcgatatgg caccacaaca attatgggtt cattcttatc ttacagaact attagaagac
841 aatgatattg ttttaattgg acctagaaaa tatgtggata ctcataatat taccgcagaa
901 caattcctta acgatccata tttaatagaa tcactacctg aaaccgctac aaataacaat
961 ccttcgatta catcaaaagg aaatatatcg ttggattgga gattagaaca tttcaaaaaa
1021 accgataatc tacgtctatg tgattctccg tttcgttatt ttagttgcgg taatgttgca
1081 ttttctaaag aatggctaaa taaagtaggt tggttcgatg aagaatttaa tcattggggg
1141 ggcgaagatg tagaatttgg ttacagatta tttgccaaag gctgtttttt cagagtaatt
1201 gacggcggaa tggcatacca tcaagaacca cctggtaaag aaaatgaaac agaccgcgaa
1261 gctggtaaaa gtattacgct taaaattgtg aaagaaaagg taccttacat ctatagaaag
1321 cttttaccaa tagaagattc acatattcat agaatacctt tagtttctat ttatatcccc
1381 gcttataact gtgcaaatta tattcaaaga tgtgtagata gtgctcttaa tcaaactgtt
1441 gtcgatctcg aggtttgtat ttgtaacgat ggttcaacag ataatacctt agaagtgatc
1501 aataagcttt atggtaataa tcctagggta cgcatcatgt ctaaaccaaa tggcggaata
1561 gcctcagcat caaatgcagc cgtttctttt gctaaaggtt attacattgg gcagttagat
1621 tcagatgatt atcttgagcc tgatgcagtt gaactgtgtt taaaagaatt tttaaaagat
1681 aaaacgctag cttgtgttta taccactaat agaaacgtca atccggatgg tagcttaatc
1741 gctaatggtt acaattggcc agaattttca cgagaaaaac tcacaacggc tatgattgct
1801 caccatttta gaatgtttac gattagagct tggcatttaa cggatggatt taacgaaaat
1861 attgaaaacg ccgtggatta tgacatgttc cttaaactca gtgaagttgg aaaatttaaa
1921 catcttaata aaatctgcta taaccgcgta ttacatggtg ataacacatc cattaagaaa
1981 ctcggcattc aaaagaaaaa ccattttgtt gtagtcaatc agtcattaaa tagacaaggc
2041 atcaattatt ataattatga caaatttgat gatttagatg aaagtagaaa gtatatcttc
2101 aataaaaccg ctgaatatca agaagaaatg gatattttaa aagatcttaa actcattcaa
2161 aataaagatg ccaaaatcgc agtcagtatt ttctatccca atacattaaa cggcttagtg
2221 aaaaaactaa acaatattat tgaatataat aaaaatatat tcgttattat tctacatgtt
2281 gataagaatc atcttacacc agacatcaaa aaagaaatat tggctttcta tcataagcac
2341 caagtgaata ttttactaaa taatgacatc tcatattaca cgagtaatag actaataaaa
2401 actgaggcac atttaagtaa tattaataaa ttaagtcagt taaatctaaa ttgtgaatac
2461 atcatttttg ataatcatga cagcctattc gttaaaaatg acagctatgc ttatatgaaa
2521 aaatatgatg tcggcatgaa tttctcagca ttaacacatg attggatcga gaaaatcaat
 
2581 gcgcatccac catttaaaaa gctgattaaa acctatttta atgacaatga cttaagaagt
2641 atgaatgtga aaggggcatc acaaggtatg tttatgaagt atgcgctacc gcatgagctt
2701 ctgacgatta ttaaagaagt catcacatcc tgccaatcaa ttgatagtgt gccagaatat
2761 aacactgagg atatttggtt ccaatttgca cttttaatct tagaaaagaa aaccggccat
2821 gtatttaata aaacatcgac cctgacttat atgccttggg aacgaaaatt acaatggaca
2881 aatgaacaaa ttcaaagtgc aaaaaaaggc gaaaatatcc ccgttaacaa gttcattatt
2941 aatagtataa cgctataaaa catttgcatt ttattaaaa
<110> Paul DeAngelis
<120> CHONDROITIN SYNTHASE GENE AND METHODS OF MAKING AND USING SAME
<130> 5820.601
<140> Not. Yet Assigned
<141> Herewith
<160> 7
<170> WordPerfect 8.0 *Software*
<210> 4.
<211> 965 amino acids
<212> PRT
<213> Pasteurella Multocida
<220>
<400> pmCS Protein Sequence 
1 mmtlsqaika ynsndyelal klfeksaety grkivefqii kckeklstns yvsedkknsv
61 cdssldiatq lllsnvkklt lseseknslk nkwksitgkk senaeirkve lvpkdfpkdl
121 vlaplpdhvn dftwyknrkk slgikpvnkn iglsiiiptf nrsrilditl aclvnqktny
181 pfevvvaddg skenlltivq kyeqkldiky vrqkdygyql cavrnlglrt akydfvsild
241 cdmapqqlwv hsyltelled ndivligprk yvdthnitae qflndpylie slpetatnnn
301 psitskgnis ldwrlehfkk tdnlrlcdsp fryfscgnva fskewlnkvg wfdeefnhwg
361 gedvefgyrl fakgcffrvi dggmayhqep pgkenetdre agksitlkiv kekvpyiyrk
421 llpiedshih riplvsiyip ayncanyiqr cvdsalnqtv vdlevcicnd gstdntlevi
481 nklygnnprv rimskpnggi asasnaavsf akgyyigqld sddylepdav elclkeflkd
541 ktlacvyttn rnvnpdgsli angynwpefs reklttamia hhfrmftira whltdgfnen
601 ienavdydmf lklsevgkfk hlnkicynrv lhgdntsikk lgiqkknhfv vvnqslnrgg
661 inyynydkfd dldesrkyif nktaeyqeem dilkdlkliq nkdakiavsi fypntlnglv
721 kklnniieyn knifviilhv dknhltpdik keilafyhkh qvnillnndi syytsnrlik
781 teahlsnink lsqlnlncey iifdnhdslf vkndsyaymk kydvgmnfsa lthdwiekin
841 ahppfkklik tyfndndlrs mnvkgasqgm fmkyalphel ltiikevits cqsidsvpey
901 ntediwfqfa llilekktgh vfnktstlty mpwerklqwt neqiqsakkg enipvnkfii
961 nsitl
<110> Paul DeAngelis
<120> CHONDROITIN SYNTHASE GENE AND METHODS OF MAKING AND USING SAME
<130> 5820.601
<140> Not Yet Assigned
<141> Herewith
<160> 7
<170> WordPerfect 8.0 *Software*
<210> 5
<211> 24 base pairs
<212> DNA
<213> Pasteurella Multocida
<220>
<400> Primer
   CACTGTCTAACTTTATTGTTAGCC
<110> Paul DeAngelis
<120> CHONDROITIN SYNTHASE GENE AND METHODS OF MAKING AND USING SAME
<130> 5820.601
<140> Not Yet Assigned
<141> Herewith
<160> 7
<170> WordPerfect 8.0 *Software*
<210> 6
<211> 20 base pairs
<212> DNA
<213> Pasteurella Multocida
<220>
<400> Primer
   TTTTTAACGAATAGGCTGTC
<110> Paul DeAngelis
<120> CHONDROITIN SYNTHASE GENE AND METHODS OF MAKING AND USING SAME
<130> 5820.601
<140> Not Yet Assigned
<141> Herewith
<160> 7
<170> WordPerfect 8.0 *Software*
<210> 7
<211> 19 amino acids
<212> PRT
<213> Pasteurella Multocida
<220>
<400> Antibody
   LDSDDYLEPDAVELCLKEF

## Claims

1. A purified nucleic acid segment consisting of a coding region encoding enzymatically active soluble chondroitin synthase, wherein the purified nucleic acid segment is selected from the group consisting of:
(A) a purified nucleic acid segment corresponding to residues 45 to 704 of SEQ ID NO:3;
(B) a purified nucleic acid segment corresponding to residues 75 to 704 of SEQ ID NO:3; and
(C) a purified nucleic acid segment corresponding to residues 1 to 704 of SEQ ID NO:3.

2. A recombinant vector selected from the group consisting of a plasmid, cosmid, phage, integrated cassette or virus vector and wherein the recombinant vector further comprises the purified nucleic acid segment of claim 1.

3. The recombinant vector of claim 2, wherein the plasmid further comprises an expression vector.

4. The recombinant vector of claim 3, wherein the expression vector comprises a promoter operatively linked to the enzymatically active *Pasteurella multocida* soluble chondroitin synthase coding region.

5. A recombinant host cell, wherein the recombinant host cell is a prokaryotic or eukaryotic cell electroporated, transduced or transformed with the recombinant vector of any of claims 2-4, wherein the eukaryotic cell is neither a human germ cell nor an embryonic stem cell.

6. The recombinant host cell of claim 5, wherein the host cell produces chondroitin.

7. The recombinant host cell of claim 5, wherein the enzymatically active chondroitin synthase is capable of producing a chondroitin polymer having a modified structure.

8. The recombinant host cell of claim 5, wherein the enzymatically active chondroitin synthase is capable of producing a chondroitin polymer having a modified size distribution.

9. The recombinant host cell of any of claims 5-8, further containing at least one of an epimerase and a sulfotransferase.

10. A method for producing a chondroitin polymer *in vitro* comprising the steps of:
- providing a chondroitin synthase, wherein the chondroitin synthase is encoded by the purified nucleic acid segment of claim 1;
- placing the chondroitin synthase in a medium suitable for the expression of a chondroitin polymer; and
- extracting the chondroitin polymer out of the medium.

11. A method for producing a chondroitin polymer *in vivo,* comprising the steps of:
- providing a purified nucleic acid segment encoding chondroitin synthase, wherein the chondroitin synthase is encoded by the nucleic acid segment of claim 1;
- placing the purified nucleic acid segment encoding chondroitin synthase in a native or recombinant prokaryotic organism, thereby providing a native or recombinant organism having a chondroitin synthase therein;
- placing the native or recombinant organism having a chondroitin synthase therein in a medium suitable for the expression of a chondroitin polymer; and
- extracting the chondroitin polymer.

12. The method of claim 11, wherein the native or recombinant prokaryotic organism contains nucleic acid segments encoding enzymes which produce UDP-GlcUA and UDP-GalNAc.

13. The method according to claim 11, wherein in the step of recovering the chondroitin polymer, the chondroitin polymer is extracted from the medium or the cells or combinations thereof.

14. The method according to claim 13, further comprising the steps of purifying the extracted chondroitin polymer.

15. The method according to claim 11, further comprising the step of sulfating the chondroitin polymer.

16. The method according to claim 11, further comprising the step of epimerizing the chondroitin polymer.

## Patentansprüche

1. Gereinigtes Nucleinsäuresegment, bestehend aus einer codierenden Region, die enzymatisch aktive, lösliche Chondroitinsynthase codiert, wobei das gereinigte Nucleinsäuresegment ausgewählt ist aus der Gruppe, bestehend aus:
(A) einem gereinigten Nucleinsäuresegment, das den Resten 45 bis 704 der SEQ ID-Nr. 3 entspricht;
(B) einem gereinigten Nucleinsäuresegment, das den Resten 75 bis 704 der SEQ ID-Nr. 3 entspricht und
(C) einem gereinigten Nucleinsäuresegment, das den Resten 1 bis 704 der SEQ ID-Nr. 3 entspricht.

2. Rekombinationsvektor, der ausgewählt ist aus der Gruppe, bestehend aus einem Plasmid, einem Cosmid, einer Phage, einer integrierten Kassette oder einem Virusvektor, und wobei der Rekombinationsvektor ferner das Nucleinsäuresegment nach Anspruch 1 aufweist.

3. Rekombinationsvektor nach Anspruch 2, wobei das Plasmid ferner einen Expressionsvektor aufweist.

4. Rekombinationsvektor nach Anspruch 3, wobei der Expressionsvektor einen Promotor aufweist, der operativ verbunden ist mit der die enzymatisch aktive *Pasteurella multocida* löslichen Chondroitin-Synthase codierenden Region.

5. Rekombinante Wirtszelle, wobei die rekombinante Wirtszelle eine prokaryotische oder eine eukaryotische Zelle ist, die mit dem Rekombinationsvektor nach einem der Ansprüche 2 bis 4 elektroporiert, transduziert oder transformiert wurde, wobei die eukaryotische Zelle weder eine Keimzelle des Menschen ist noch eine embryonische Stammzelle ist.

6. Rekombinante Wirtszelle nach Anspruch 5, wobei die Wirtszelle Chondroitin erzeugt.

7. Rekombinante Wirtszelle nach Anspruch 5, wobei die enzymatisch aktive Chondroitin-Synthase zum Erzeugen von Polymer des Chondroitins in der Lage ist, das über eine modifizierte Struktur verfügt.

8. Rekombinante Wirtszelle nach Anspruch 5, wobei die enzymatisch aktive Chondroitin-Synthase zum Erzeugen von Polymer des Chondroitins in der Lage ist, das über eine modifizierte Größenverteilung verfügt.

9. Rekombinante Wirtszelle nach einem der Ansprüche 5 bis 8, ferner mindestens enthaltend eine Epimerase oder eine Sulfotransferase.

10. Verfahren zum Erzeugen eines Polymers von Chondroitin *in vitro,* welches Verfahren die Schritte umfasst:
- Bereitstellen einer Chondroitinssynthase, wobei die Chondroitin-synthase mit Hilfe des gereinigten Nucleinsäuresegments nach Anspruch 1 codiert ist;
- die Chondroitinsynthase in ein Medium bringen, das für die Expression eines Polymers von Chondroitin in der Lage ist, und
- das Polymer von Chondroitin aus dem Medium extrahieren.

11. Verfahren zum Erzeugen eines Polymers von Chondroitin *in vivo,* welches Verfahren die Schritte umfasst:
- Bereitstellen eines gereinigten Nucleinsäuresegments, welches Chondroitinsynthase codiert, wobei die Chondroitinsynthase durch das Nucleinsäuresegment nach Anspruch 1 codiert wird;
- das gereinigte Nucleinsäuresegment, welches Chondroitinsynthase codiert, in einen nativen oder rekombinanten prokaryotischen Organismus bringen, wodurch ein nativer oder rekombinanter Organismus bereitgestellt wird, der darin über eine Chondroitinsynthase verfügt;
- den nativen oder rekombinanten Organismus, der darin über eine Chondroitinsynthase verfügt, in ein Medium bringen, das für die Expression eines Polymers von Chondroitin geeignet ist, und
- Extrahieren des Polymers von Chondroitin.

12. Verfahren nach Anspruch 11, bei welchem der native oder rekombinante prokaryotische Organismus Nucleinsäuresegmente enthält, die Enzyme codieren, welche UDP-GlcUA und UDP-GalNAc erzeugen.

13. Verfahren nach Anspruch 11, bei welchem in dem Schritt der Gewinnung des Polymers von Chondroitin das Polymer des Chondroitins aus dem Medium oder den Zellen oder Kombinationen davon extrahiert wird.

14. Verfahren nach Anspruch 13, ferner umfassend die Schritte des Reinigens des extrahierten Polymers von Chondroitin.

15. Verfahren nach Anspruch 11, ferner umfassend den Schritt des Sulfatierens des Polymers von Chondroitin.

16. Verfahren nach Anspruch 11, ferner umfassend den Schritt des Epimerisierens des Polymers von Chondroitin.

## Revendications

1. Segment d'acide nucléique purifié constitué d'une région codante codant pour de la chondroïtine synthase soluble active par voie enzymatique, dans lequel le segment d'acide nucléique purifié est sélectionné parmi le groupe constitué de :
(A) un segment d'acide nucléique purifié correspondant aux résidus 45 à 704 de SEQ ID N°:3;
(B) un segment d'acide nucléique purifié correspondant aux résidus 75 à 704 de SEQ ID N° : 3 ; et
(C) un segment d'acide nucléique purifié correspondant aux résidus 1 à 704 de SEQ ID N° : 3.

2. Vecteur recombiné sélectionné parmi le groupe constitué d'un plasmide, d'un cosmide, d'un bactériophage, d'une cassette ou d'un vecteur viral intégré et dans lequel le vecteur recombiné comprend en outre le segment d'acide nucléique purifié selon la revendication 1.

3. Vecteur recombiné selon la revendication 2, dans lequel le plasmide comprend en outre un vecteur d'expression.

4. Vecteur recombiné selon la revendication 3, dans lequel le vecteur d'expression comprend un promoteur lié de manière opérationnelle à la région codante de la chondroïtine synthase soluble active par voie enzymatique avec Pasteurella multocida.

5. Cellule hôte recombinée, dans laquelle la cellule hôte recombinée est une cellule procaryote ou eucaryote ayant subi une électroporation, transduction ou transformation avec le vecteur recombiné selon l'une quelconque des revendications 2 à 4, dans laquelle la cellule eucaryote n'est ni une cellule germinale humaine, ni une cellule souche embryonnaire.

6. Cellule hôte recombinée selon la revendication 5, dans laquelle la cellule hôte produit de la chondroïtine.

7. Cellule hôte recombinée selon la revendication 5, dans laquelle la chondroïtine synthase active par voie enzymatique est capable de produire un polymère de chondroïtine ayant une structure modifiée.

8. Cellule hôte recombinée selon la revendication 5, dans laquelle la chondroïtine synthase active par voie enzymatique est capable de produire un polymère de chondroïtine ayant une distribution de taille modifiée.

9. Cellule hôte recombinée selon l'une quelconque des revendications 5 à 8, contenant en outre au moins une enzyme parmi une épimérase et une sulfotransférase.

10. Procédé de production d'un polymère de chondroïtine in vitro, comprenant les étapes consistant à :
- fournir une chondroïtine synthase, dans lequel la chondroïtine synthase est codée par le segment d'acide nucléique purifié selon la revendication 1 ;
- placer la chondroïtine synthase dans un milieu approprié pour l'expression d'un polymère de chondroïtine ; et
- extraire le polymère de chondroïtine du milieu.

11. Procédé de production d'un polymère de chondroïtine in vivo, comprenant les étapes consistant à :
- fournir un segment d'acide nucléique purifié codant pour la chondroïtine synthase, dans lequel la chondroïtine synthase est codée par le segment d'acide nucléique selon la revendication 1 ;
- placer le segment d'acide nucléique purifié codant pour la chondroïtine synthase dans un organisme procaryote natif ou recombiné, fournissant de ce fait un organisme natif ou recombiné ayant une chondroïtine synthase en son sein ;
- placer l'organisme natif ou recombiné ayant une chondroïtine synthase en son sein dans un milieu approprié pour l'expression d'un polymère de chondroïtine ; et
- extraire le polymère de chondroïtine.

12. Procédé selon la revendication 11, dans lequel l'organisme procaryote natif ou recombiné contient des segments d'acide nucléique codant pour des enzymes qui produisent UDP-GlcUA et UDP-GalNAc.

13. Procédé selon la revendication 11, dans lequel dans l'étape de récupération du polymère de chondroïtine, le polymère de chondroïtine est extrait du milieu ou des cellules ou de combinaisons de ceux-ci.

14. Procédé selon la revendication 13, comprenant en outre les étapes de purification du polymère de chondroïtine extrait.

15. Procédé selon la revendication 11, comprenant en outre l'étape de sulfatation du polymère de chondroïtine.

16. Procédé selon la revendication 11, comprenant en outre l'étape d'épimérisation du polymère de chondroïtine.
